# EUROPEAN PATENT APPLICATION

(11) **EP 4 725 302 A2**
(43) Date of publication of application: **15.04.2026**
(21) Application number: 25204031.6
(22) Date of filing: 23.09.2025
(51) Int. Cl.: A01H 1/04, A01H 1/00, A01H 5/10, A01H 6/54

(54) **COMPOSITIONS AND METHODS FOR PRODUCING PEA PLANTS WITH ENHANCED YIELD, HEIGHT, AND/OR LODGING PHENOTYPE**

(30) Priority: 23.09.2024 US 202463697851 P
(71) Applicant: Confluence Genetics, LLC, St. Louis, MO 63132 (US)
(72) Inventor: Goettel, Herbert Wolfgang, St. Louis 63132 (US); Kofsky, Janice R., St. Louis 63132 (US)
(74) Representative: Inspicos P/S

(57) **Abstract**

Provided herein are methods and compositions for producing pea plants having a desirable yield, height, and/or lodging phenotype using marker-assisted selection. The disclosure further provides methods and compositions for introgressing one or more loci comprising at least an allele linked to the yield, height, and/or lodging QTL, thus producing pea plants with a desirable yield, height, and/or lodging phenotype.

## Description

### CROSS REFERENCE TO RELATED APPLICATIONS

This application claims priority to U.S. Provisional Application No. 63/697,851 filed on September 23, 2024, the content of which is incorporated herein by reference in its entirety.

### SEQUENCE LISTING

This application contains a Sequence Listing which is submitted herewith in electronically readable format. The Sequence Listing file was created on September 20, 2025, is named "B88552_1660_SL.xml" and its size is 177320 bytes. The entire contents of the Sequence Listing file are incorporated by reference herein.

### FIELD OF THE INVENTION

This disclosure relates generally to the field of agricultural biotechnology. More specifically, this disclosure relates to methods for producing pea plants or seeds with a desirable yield, height, and/or lodging phenotype. Also provided herein are compositions for use in such methods.

### BACKGROUND OF THE INVENTION

Pea is an excellent source of nutrients (e.g., protein, sugar, oil) and supplies adequate and nutritious food and feed compositions. Most commercially produced peas are processed to produce edible oil and/or protein products for human and animal consumption. Pea compositions can also be used to prepare products such as enzymes, pectin, nanoparticles, and polymers for industrial applications. There is a great need to obtain pea varieties that possess a desirable yield, height, and/or lodging phenotype. Peas with a desirable yield, height, and/or lodging phenotype (e.g., high yield and/or low lodging, optionally in combination with high height or low height) would represent a substantial commercial value that would benefit producers, processors, and consumers alike.

### SUMMARY OF THE INVENTION

The present disclosure identifies genetic loci conferring desirable yield, height, and/or lodging phenotype in pea, and provides molecular markers linked to these yield, height, and/or lodging loci. The yield, height, and lodging phenotypes can be associated, and the markers linked to the yield, height, and/or lodging genetic loci may coincide. This disclosure provides methods of producing a population of pea plants or seeds with a desirable yield, height, and/or lodging phenotype. Further provided are methods of introgressing a yield, height, and/or lodging QTL, thereby a progeny plant or seed comprising a yield, height, and/or lodging allele of a polymorphic locus linked to the yield, height, and/or lodging QTL. The genetic loci, markers, and methods provided herein therefore allow for production of new varieties of pea plants with a desirable yield, height, and/or lodging phenotype.

Accordingly, in a first aspect, provided herein is a method of producing a population of pea plants or seeds with a desirable yield, height, and/or lodging phenotype. The method includes a) genotyping a first population of pea plants or seeds for the presence of at least one yield, height, and/or lodging molecular marker that is within 20 centimorgans of one or more yield, height, and/or lodging Quantitative Trait Locus (QTLs) selected from the group consisting of yield chr2-2 (Ps02_417306163), yield chr5-1 (Ps05_117545329), yield chr5-2 (Ps05_604790207), lodging chr2-1 (Ps02_415727951), lodging chr5-1 (Ps05_608256598), lodging chr6-1 (Ps06_5054240), height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364; b) selecting from the first population one or more pea plants or seeds comprising one or more yield, height, and/or lodging alleles having the at least one yield, height, and/or lodging molecular marker; and c) producing a second population of progeny pea plants or seeds from the selected one or more pea plants or plants grown from the selected seeds, wherein the second population of progeny pea plants or seeds comprises the one or more yield, height, and/or lodging alleles having the at least one yield, height, and/or lodging molecular marker. The progeny pea plants or seeds of the second population are pea plants or seeds with a desirable yield, height, and/or lodging phenotype. Thus, the method produces a population of pea plants or seeds with a desirable yield, height, and/or lodging phenotype.

In some embodiments of the method provided herein of producing a population of pea plants or seeds with a desirable yield, height, and/or lodging phenotype, at least one yield, height, and/or lodging molecular marker is within 10 centimorgans of said one or more yield, height, and/or lodging QTLs.

In some embodiments of the method provided herein of producing a population of pea plants or seeds with a desirable yield, height, and/or lodging phenotype, one or more yield, height, and/or lodging QTLs are selected from the group consisting of Ps02_406013484, Ps02_415945622, yield chr2-2 (Ps02_417306163), height chr2-2 (Ps2_390480167), lodging chr2-1 (Ps02_415727951), Ps06_32259152, Ps06_ 43753843, height chr6-1 (Ps06_38889673), Ps07_129103458, height chr7-2 (Ps07_129458029), Ps03_71303964, height chr3-1 (Ps03_74158043), Ps05_583707944, Ps05_612824899, Ps05_616716582, lodging chr5-1 (Ps05_608256598), yield chr5-2 (Ps05_604790207), Ps06_12615016, Ps06_33377536, height chr6-1 (Ps06_38889673), Ps07_133823647, Ps07_112377551, height chr7-1 (Ps07_115631962), Ps04_205866569, height chr4-1 (Ps04_192115819), Ps07_114916793, Ps07_234681662, Ps07_238767894, and height chr7-3 (Ps07_256289873).

In some embodiments of the method provided herein of producing a population of pea plants or seeds with a desirable yield, height, and/or lodging phenotype, one or more yield, height, and/or lodging QTLs are selected from the group consisting of Ps02_406013484, Ps02_415945622, yield chr2-2 (Ps02_417306163), height chr2-2 (Ps2_390480167), lodging chr2-1 (Ps02_415727951), Ps05_583707944, Ps05_612824899, lodging chr5-1 (Ps05_608256598), yield chr5-2 (Ps05_604790207), Ps07_112377551, height chr7-1 (Ps07_115631962), Ps04_205866569, height chr4-1 (Ps04_192115819), Ps07_114916793, Ps07_234681662, Ps07_238767894, and height chr7-3 (Ps07_256289873).

In some embodiments of the method provided herein of producing a population of pea plants or seeds with a desirable yield, height, and/or lodging phenotype, one or more yield, height, and/or lodging QTLs are selected from the group consisting of Ps02_406013484, Ps03_531239107, Ps04_445153308, and Ps07_9801763.

In some embodiments of the method provided herein of producing a population of pea plants or seeds with a desirable yield, height, and/or lodging phenotype, one or more yield, height, and/or lodging QTLs comprises one or more yield QTLs selected from the group consisting of yield chr2-2 (Ps02_417306163), yield chr5-1 (Ps05_117545329), yield chr5-2 (Ps05_604790207), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, and Ps07_481516842.

In some embodiments of the method provided herein of producing a population of pea plants or seeds with a desirable yield, height, and/or lodging phenotype, one or more yield, height, and/or lodging QTLs comprises one or more height QTLs selected from the group consisting of height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps03_531239107, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364.

In some embodiments of the method provided herein of producing a population of pea plants or seeds with a desirable yield, height, and/or lodging phenotype, one or more yield, height, and/or lodging QTLs comprises one or more lodging QTLs selected from the group consisting of lodging chr2-1 (Ps02_415727951), lodging chr5-1 (Ps05_608256598), lodging chr6-1 (Ps06_5054240), Ps02_406013484, Ps04_445153308, Ps07_9801763, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515.

In some embodiments of the method provided herein of producing a population of pea plants or seeds with a desirable yield, height, and/or lodging phenotype, genotyping includes assaying a single nucleotide polymorphism (SNP) marker.

In some embodiments of the method provided herein of producing a population of pea plants or seeds with a desirable yield, height, and/or lodging phenotype, genotyping includes the use of an oligonucleotide probe or a pair of primers. In some embodiments of the method provided herein of producing a population of pea plants or seeds with a desirable yield, height, and/or lodging phenotype, the oligonucleotide probe or the pair of primers comprise a nucleic acid molecule that comprises at least 15 nucleotides that include or are immediately adjacent to the SNP or the deletion sequence, wherein the nucleic acid molecule is at least 90 percent identical to a sequence of the same number of consecutive nucleotides in either strand of DNA that include or are immediately adjacent to the SNP or the deletion sequence.

In some embodiments of the method provided herein of producing a population of pea plants or seeds with a desirable yield, height, and/or lodging phenotype, genotyping includes detecting a haplotype.

In some embodiments of the method provided herein of producing a population of pea plants or seeds with a desirable yield, height, and/or lodging phenotype, the population of pea plants or seeds having one or more yield, height, and/or lodging alleles has average yield that is greater by at least 13 bushels per acre, average height that is greater by at least 8 inches, and/or average lodging that is lower by at least 1.5 lodging units relative to a control population of pea plants or seeds without said one or more yield, height, and/or lodging alleles.

In some embodiments of the method provided herein of producing a population of pea plants or seeds with a desirable yield, height, and/or lodging phenotype, the population of pea plants or seeds having one or more yield, height, and/or lodging alleles has average protein content or average oil content that is at least 90% relative to a control population of pea plants or seeds without said one or more yield, height, and/or lodging alleles.

In some embodiments of the method provided herein of producing a population of pea plants or seeds with a desirable yield, height, and/or lodging phenotype, the method further includes determining the yield, height, and/or lodging of the second population of pea plants or seeds, wherein the second population of pea plants or seeds having the one or more yield, height, and/or lodging alleles have greater yield, greater height, and/or less lodging when compared to a control population of pea plants or seeds lacking the one or more yield, height, and/or lodging alleles. In some embodiments of the method provided herein of producing a population of pea plants or seeds with a desirable yield, height, and/or lodging phenotype, the second population of pea plants has a greater frequency of the at least one yield, height, and/or lodging molecular marker than said first population of pea plants.

In another aspect, provided herein is a methods of introgressing a yield, height, and/or lodging QTL. The method includes (a) crossing a first pea plant comprising a yield, height, and/or lodging QTL with a second pea plant of a different genotype to produce one or more progeny plants or seeds; and (b) selecting a progeny plant or seed comprising one or more yield, height, and/or lodging alleles of a polymorphic locus linked to the yield, height, and/or lodging QTL. The polymorphic locus corresponds to a chromosomal segment comprising any marker within a genomic region between positions: 386683682-424059129 of chromosome 2; 57407964-130197960 of chromosome 5, 541653217-end of chromosome 5, 387250594-427410060 of chromosome 2; 547677746-end of chromosome 5; start-17888751 of chromosome 6; 354324649-402078389 of chromosome 2; 18873917-69320372 of chromosome 3; 273327332-422042733 of chromosome 3; 118944413-200876233 of chromosome 4; 17641704-56905305 of chromosome 6; 79787890-153689044 of chromosome 7; 91738904-165762735 of chromosome 7; 207998578-360550624 of chromosome 7; scaffold00011; 73778015-347862320 of chromosome 1 LG6; 287196406-405820442 of chromosome 2 LG1; scaffold05541; 22316149-374998060 of chromosome 4 LG4; 35018291-496475925 of chromosome 5 LG3; 4102 if scaffold00839; 332130985-426328693 of chromosome 6 LG2; 8316115-481251260 of chromosome 7 LG7; scaffold02229; 122338217-340372022 of chromosome 1 LG6; 12117951-41766109 of chromosome 2 LG1; 55052537-420398638 of chromosome 3 LG5; scaffold06018; scaffold03592; super-scaffold8606; 47145729-440853776 of chromosome 4 LG4; scaffold02661; 3579419-570360377 of chromosome 5LG3; super-scaffold9678; 2560210-348314640 of chromosome 6 LG2; 191955823-63397608 of chromosome 7 LG7; 63453702 of chromosome 1 LG6; 96628783-415226641 of chromosome 2 LG1; scaffold05147; 403366757 of chromosome 4 LG4; scaffold00254; 811842-294741093 of chromosome 3 LG5; scaffold03249; 173468835-440508849 of chromosome 4 LG4; 362378-229071963 of chromosome 5 LG3; scaffold01526; 157000374-469077464 of chromosome 6 LG2; scaffold01649; or 750954-487220871 of chromosome 7 LG7 of a *Pisum sativum* genome.

In some embodiments of the method provided herein of introgressing a yield, height, and/or lodging QTL, the genomic region corresponds to a genomic region between positions: 386683682-424059129 of chromosome 2; 57407964-130197960 of chromosome 5; 541653217-end of chromosome 5; 387250594-427410060 of chromosome 2; 547677746-end of chromosome 5; start-17888751 of chromosome 6; 354324649-402078389 of chromosome 2; 18873917-69320372 of chromosome 3; 273327332-422042733 of chromosome 3; 118944413-200876233 of chromosome 4; 17641704-56905305 of chromosome 6; 79787890-153689044 of chromosome 7; 91738904-165762735 of chromosome 7; 207998578-360550624 of chromosome 7; scaffold00011; 73778015-347862320 of chromosome 1 LG6; 287196406-405820442 of chromosome 2 LG1; scaffold05541; 22316149-374998060 of chromosome 4 LG4; 35018291-496475925 of chromosome 5 LG3; 4102 if scaffold00839; 332130985-426328693 of chromosome 6 LG2; 8316115-481251260 of chromosome 7 LG7; scaffold02229; 122338217-340372022 of chromosome 1 LG6; 12117951-41766109 of chromosome 2 LG1; 55052537-420398638 of chromosome 3 LG5; scaffold06018; scaffold03592; super-scaffold8606; 47145729-440853776 of chromosome 4 LG4; scaffold02661; 3579419-570360377 of chromosome 5LG3; super-scaffold9678; 2560210-348314640 of chromosome 6 LG2; 191955823-63397608 of chromosome 7 LG7; 63453702 of chromosome 1 LG6; 96628783-415226641 of chromosome 2 LG1; scaffold05147; 403366757 of chromosome 4 LG4; scaffold00254; 811842-294741093 of chromosome 3 LG5; scaffold03249; 173468835-440508849 of chromosome 4 LG4; 362378-229071963 of chromosome 5 LG3; scaffold01526; 157000374-469077464 of chromosome 6 LG2; scaffold01649; or 750954-487220871 of chromosome 7 LG7 of the *Pisum sativum* Cameor vla reference genome.

In some embodiments of the method provided herein of introgressing a yield, height, and/or lodging QTL, the yield QTL contains an SNP marker associated with high yield; the height QTL contains an SNP marker associated with height; and the lodging QTL contains an SNP marker associated with low lodging.

In some embodiments of the method provided herein of introgressing a yield, height, and/or lodging QTL, the SNP marker is selected from the group consisting of yield chr2-2 (Ps02_417306163), yield chr5-1 (Ps05_117545329), yield chr5-2 (Ps05_604790207), lodging chr2-1 (Ps02_415727951), lodging chr5-1 (Ps05_608256598), lodging chr6-1 (Ps06_5054240), height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_ 427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_ 425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364. In some embodiments of the method provided herein of introgressing a yield, height, and/or lodging QTL, the SNP marker is selected from the group consisting of: T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; G at position 195183596 of chromosome 7LG7; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of the *Pisum sativum* genome, such as the *Pisum sativum* Cameor v1a reference genome.

In some embodiments of the method provided herein of introgressing a yield, height, and/or lodging QTL, the SNP marker is selected from the group consisting of Ps02_406013484, Ps02_415945622, yield chr2-2 (Ps02_417306163), height chr2-2 (Ps2_390480167), lodging chr2-1 (Ps02_415727951), Ps06_32259152, Ps06_ 43753843, height chr6-1 (Ps06_38889673), Ps07_129103458, height chr7-2 (Ps07_129458029), Ps03_71303964, height chr3-1 (Ps03_74158043), Ps05_583707944, Ps05_612824899, Ps05_616716582, lodging chr5-1 (Ps05_608256598), yield chr5-2 (Ps05_604790207), Ps06_12615016, Ps06_33377536, height chr6-1 (Ps06_38889673), Ps07_133823647, Ps07_112377551, height chr7-1 (Ps07_115631962), Ps04_205866569, height chr4-1 (Ps04_192115819), Ps07_114916793, Ps07_234681662, Ps07_238767894, and height chr7-3 (Ps07_256289873). In some embodiments of the method provided herein of introgressing a yield, height, and/or lodging QTL, the SNP marker is selected from the group consisting of: C at position 397000005 of chromosome 2LG1; G at position 405820442 of chromosome 2LG1; T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T or C at position 31021563 of chromosome 6LG2; A at position 132484304 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; T at position 543282804 of chromosome 5LG3; T at position 570360377 of chromosome 5LG3; C at position 8937964 of chromosome 5LG3; G at position 566655216 of chromosome 5; C at position 563627520 of chromosome 5; A at position 11907302 of chromosome 6LG2; A at position 2560210 of chromosome 6LG2; T or C at position 31021563 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A or G at position 45871371 of chromosome 7LG7; A or G at position 115361106 of chromosome 7LG7; T or G at position 173468835 of chromosome 4LG4; T or C at position 158451622 of chromosome 4; T at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; A or G at position 116467927 of chromosome 7LG7; C or T at position 293412012 of chromosome 7LG7; T or G at position 310516174 of chromosome 7LG7; A or G at position 324031049 of chromosome 7; A or G at position 13857 of scaffold 04080; and A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity of the *Pisum sativum* genome such as the *Pisum sativum* Cameor v1a reference genome.

In some embodiments of the method provided herein of introgressing a yield, height, and/or lodging QTL, the SNP marker is selected from the group consisting of Ps02_406013484, Ps02_415945622, yield chr2-2 (Ps02_417306163), height chr2-2 (Ps2_390480167), lodging chr2-1 (Ps02_415727951), Ps05_583707944, Ps05_612824899, lodging chr5-1 (Ps05_608256598), yield chr5-2 (Ps05_604790207), Ps07_112377551, height chr7-1 (Ps07_115631962), Ps04_205866569, height chr4-1 (Ps04_192115819), Ps07_114916793, Ps07_234681662, Ps07_238767894, and height chr7-3 (Ps07_256289873). In some embodiments of the method provided herein of introgressing a yield, height, and/or lodging QTL, the SNP marker is selected from the group consisting of: C at position 397000005 of chromosome 2LG1; G at position 405820442 of chromosome 2LG1; T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; T at position 543282804 of chromosome 5LG3; T at position 570360377 of chromosome 5LG3; G at position 566655216 of chromosome 5; C at position 563627520 of chromosome 5; A or G at position 45871371 of chromosome 7LG7; A or G at position 115361106 of chromosome 7LG7; T or G at position 173468835 of chromosome 4LG4; T or C at position 158451622 of chromosome 4; T at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; A or G at position 116467927 of chromosome 7LG7; C or T at position 293412012 of chromosome 7LG7; T or G at position 310516174 of chromosome 7LG7; A or G at position 324031049 of chromosome 7; A or G at position 13857 of scaffold 04080; and A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity of the *Pisum sativum* genome such as the *Pisum sativum* Cameor v1a reference genome.

In some embodiments of the method provided herein of introgressing a yield, height, and/or lodging QTL, the SNP marker is selected from the group consisting of Ps02_406013484, Ps03_531239107, Ps04_445153308, and Ps07_9801763. In some embodiments of the method provided herein of introgressing a yield, height, and/or lodging QTL, the SNP marker is selected from the group consisting of: C at position 397000005 of chromosome 2LG1; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; C at position 374998060 of chromosome 4LG4; and A at position 8316115 of chromosome 7LG7 of the *Pisum sativum* genome such as the *Pisum sativum* Cameor vla reference genome.

In some embodiments of the method provided herein of introgressing a yield, height, and/or lodging QTL, the SNP marker associated with high yield is selected from the group consisting of yield chr2-2 (Ps02_417306163), yield chr5-1 (Ps05_117545329), yield chr5-2 (Ps05_604790207), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763,Ps07_39866790,Ps07_129103458,Ps07_142955054,Ps07_173064631, Ps07_173321635, Ps07_278403545, Ps07_303005109, and Ps07_481516842. In some embodiments of the method provided herein of introgressing a yield, height, and/or lodging QTL, the SNP marker is selected from the group consisting of: T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; and T at position 481251260 of chromosome 7LG7 of the *Pisum sativum* genome such as the *Pisum sativum* Cameor vla reference genome.

In some embodiments of the method provided herein of introgressing a yield, height, and/or lodging QTL, the SNP marker associated with height is selected from the group consisting of height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps03_531239107, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364. In some embodiments of the method provided herein of introgressing a yield, height, and/or lodging QTL, the SNP marker is selected from the group consisting of: C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A or G at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of the *Pisum sativum* genome such as the *Pisum sativum* Cameor v1a reference genome.

In some embodiments of the method provided herein of introgressing a yield, height, and/or lodging QTL, the SNP marker associated with low lodging is selected from the group consisting of lodging chr2-1 (Ps02_415727951), lodging chr5-1 (Ps05_608256598), lodging chr6-1 (Ps06_5054240), Ps02_406013484, Ps04_445153308, Ps07_9801763, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, and Ps07_195412515. In some embodiments of the method provided herein of introgressing a yield, height, and/or lodging QTL, the SNP marker is selected from the group consisting of: C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C at position 397000005 of chromosome 2LG1; C at position 374998060 of chromosome 4LG4; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; and G at position 195183596 of chromosome 7LG7 of the *Pisum sativum* genome such as the *Pisum sativum* Cameor v1a reference genome.

In one aspect, provided herein is a population of pea plants or seeds produced by the method provided herein. The population of pea plants or seeds contains a greater yield, greater height, and/or less lodging phenotype relative to a control population of pea plants or seeds lacking the one or more yield, height, and/or lodging alleles.

In some embodiments of the population of pea plants or seeds provided herein, the population of pea plants or seeds has average yield that is greater by at least 13 bushels per acre, average height that is greater by at least 8 inches, and/or average lodging that is lower by at least 1.5 lodging units relative to a control population of pea plants or seeds without said one or more yield, height, and/or lodging alleles.

In some embodiments of the population of pea plants or seeds provided herein, the population of pea plants or seeds has average protein content or average oil content that is at least 90% relative to a control population of pea plants or seeds without said one or more yield, height, and/or lodging alleles.

In one aspect, provided herein is a nucleic acid molecule for detecting a yield, height, and/or lodging molecular marker in a *Pisum sativum* genome. The nucleic acid molecule contains at least 15 nucleotides that include or are immediately adjacent to the marker, wherein the nucleic acid molecule is at least 90 percent identical to a sequence of the same number of consecutive nucleotides in either strand of DNA that include or are immediately adjacent to the marker.

In some embodiments of the nucleic acid molecule provided herein, the yield, height, and/or lodging molecular marker is an SNP marker, and the SNP marker is selected from the group consisting of T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A or G at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; G at position 195183596 of chromosome 7LG7; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of the *Pisum sativum* genome such as the *Pisum sativum* Cameor v1a reference genome.

In some embodiments of the nucleic acid molecule provided herein, the position on the chromosome corresponds to the position on the *Pisum sativum* Cameor vla reference genome.

In some embodiments of the nucleic acid molecule provided herein, the nucleic acid molecule provided herein further includes a detectable label. In some embodiments of the nucleic acid molecule provided herein, the detectable label is a fluorescent label or a radioactive label.

### DETAILED DESCRIPTION OF THE INVENTION

### 1.1. References and Definitions

The present disclosure now will be described more fully hereinafter. The disclosure may be embodied in many different forms and should not be construed as limited to the aspects set forth herein; rather, these aspects are provided so that this disclosure will satisfy applicable legal requirements.

As used herein, "a," "an," or "the" can mean one or more than one. For example, "a" cell can mean a single cell or a multiplicity of cells. Further, the term "a plant" may include a plurality of plants.

As used herein, unless specifically indicated otherwise, the word "or" is used in the inclusive sense of "and/or" and not the exclusive sense of "either/or."

The term "about" or "approximately" usually means within 5%, or more preferably within 1%, of a given value or range.

The terms "comprises," "comprising," "includes," "including," "having" and their conjugates mean "including but not limited to."

Various embodiments of this disclosure may be presented in a range format. It should be noted that whenever a value or range of values of a parameter are recited, it is intended that values and ranges intermediate to the recited values are also part of this disclosure. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1-10 should be considered to have specifically disclosed subranges such as from 1 to 3, from 1 to 4, from 1 to 5, from 1 to 6, from 1 to 7, from 1 to 8, from 1 to 9, from 2 to 4, from 2 to 6, from 2 to 8, from 2 to 10, from 3 to 6, etc., as well as individual numbers within that range, for example, 1, 2, 3, 4, 5, 6, 7, 8, 9 and 10. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein, "quantitative trait locus" (QTL) or "quantitative trait loci" (QTLs) refer to a genetic domain that effects a phenotype that can be described in quantitative terms and can be assigned a "phenotypic value" which corresponds to a quantitative value for the phenotypic trait.

As used herein, "allele" refers to an alternative nucleic acid sequence at a particular locus. The length of an allele can be as small as one nucleotide base. For example, a first allele can occur on one chromosome, while a second allele occurs on a second homologous chromosome, e.g., as occurs for different chromosomes of a heterozygous individual, or between different homozygous or heterozygous individuals in a population.

As used herein, "locus" is a chromosome region or chromosomal region where a polymorphic nucleic acid, trait determinant, gene, or marker is located. A locus may represent a single nucleotide, a few nucleotides or a large number of nucleotides in a genomic region. The loci of this disclosure comprise one or more polymorphisms in a population; e.g., alternative alleles are present in some individuals. A "gene locus" is a specific chromosome location in the genome of a species where a specific gene can be found.

An allele of a QTL can, as used herein, can comprise multiple genes or other genetic factors even within a contiguous genomic region or linkage group, such as a haplotype. As used herein, an allele of a QTL can therefore encompasses more than one gene or other genetic factor where each individual gene or genetic component is also capable of exhibiting allelic variation and where each gene or genetic factor is also capable of eliciting a phenotypic effect on the quantitative trait in question. In an embodiment of the present invention the allele of a QTL comprises one or more genes or other genetic factors that are also capable of exhibiting allelic variation. The use of the term "an allele of a QTL" is thus not intended to exclude a QTL that comprises more than one gene or other genetic factor. Specifically, an "allele of a QTL" in the present in the invention can denote a haplotype within a haplotype window wherein a phenotype can be disease resistance. A haplotype window is a contiguous genomic region that can be defined, and tracked, with a set of one or more polymorphic markers wherein said polymorphisms indicate identity by descent. A haplotype within that window can be defined by the unique fingerprint of alleles at each marker. As used herein, an allele is one of several alternative forms of a gene occupying a given locus on a chromosome. When all the alleles present at a given locus on a chromosome are the same, that plant is homozygous at that locus. If the alleles present at a given locus on a chromosome differ, that plant is heterozygous at that locus.

An "effect size" as used herein in the context of QTLs refers to the variance of a phenotype explained by the QTL. The statistical definition of the effect size of a QTL may vary depending on studies. For example, the QTL effect size (h²_{QTL}) can be defined as h²_{QTL} = V(2β), where V() denotes the population variance, and β denotes the sample variance of the allele effects. Under this definition, the QTL effect size is interpretable as the variance that would be explained by the QTL in a theoretical population that is balanced with respect to the functional alleles. Alternatively, the QTL effect size (h²_{QTL}) can be defined as h²_{QTL} = V(DAMβ), where V() denotes the population variance, D denotes locus diplotypes of a particular polymorphism, A denotes the haplotype matrix, M denotes the allelic series configuration, and β denotes the sample variance of the allele effects. Under this definition, the QTL effect size is the variance (R²) of a phenotype explained by the QTL in the mapping population, and depends on both M and D.

As used herein, a "haplotype" is the genotype of an individual at a plurality of genetic loci. Typically, the genetic loci described by a haplotype are physically and genetically linked, e.g., in the same chromosome interval. A haplotype can also refer to a combination of SNP alleles located within a single gene.

As used herein, "polymorphism" means the presence of one or more variations in a population. A polymorphism may manifest as a variation in the nucleotide sequence of a nucleic acid or as a variation in the amino acid sequence of a protein. Polymorphisms include the presence of one or more variations of a nucleic acid sequence or nucleic acid feature at one or more loci in a population of one or more individuals. The variation may comprise but is not limited to one or more nucleotide base changes, the insertion of one or more nucleotides or the deletion of one or more nucleotides. A polymorphism may arise from random processes in nucleic acid replication, through mutagenesis, as a result of mobile genomic elements, from copy number variation and during the process of meiosis, such as unequal crossing over, genome duplication and chromosome breaks and fusions. The variation can be commonly found or may exist at low frequency within a population, the former having greater utility in general plant breeding and the latter may be associated with rare but important phenotypic variation. Useful polymorphisms may include single nucleotide polymorphisms (SNPs), insertions or deletions in DNA sequence (Indels), simple sequence repeats of DNA sequence (SSRs), a restriction fragment length polymorphism, and a tag SNP. A genetic marker, a gene, a DNA-derived sequence, a RNA-derived sequence, a promoter, a 5' untranslated region of a gene, a 3' untranslated region of a gene, microRNA, siRNA, a tolerance locus, a satellite marker, a transgene, mRNA, ds mRNA, a transcriptional profile, and a methylation pattern may also comprise polymorphisms. In addition, the presence, absence, or variation in copy number of the preceding may comprise polymorphisms.

As used herein, "SNP" or "single nucleotide polymorphism" means a sequence variation that occurs when a single nucleotide (A, T, C, or G) in the genome sequence is altered or variable.

As used herein, "marker," or "molecular marker," or "marker locus" is a term used to denote a nucleic acid or amino acid sequence that is sufficiently unique to characterize a specific locus on the genome

As used herein, a centimorgan ("cM") is a unit of measure of recombination frequency and genetic distance between two loci. One cM is equal to a 1% chance that a marker at one genetic locus will be separated from a marker at, a second locus due to crossing over in a single generation.

As used herein, "correspond to" or "corresponding to" with respect to genomic regions, loci, or positions indicates that the specified chromosomal region, locus, or position in the first chromosome aligns with the specified chromosomal region, locus, or position in the second chromosome when the two chromosomes are subjected to standard sequence alignments (e.g., using the BLAST program) and aligned for maximum sequence identity (such as at least 85%, 86%, 87%, 88%, 89%, 90%, 91%, 92%, 93%, 94%, 95%, 96%, 97%, 98%, 99%, or 100% sequence identity) across the entire chromosome or chromosomal scaffold segment. Thus, the polymorphic locus "X" in the first chromosome will correspond to the polymorphic locus "Y" in the second chromosome if residues X and Y correspond to each other in a sequence alignment, and despite the fact that X and Y may be at different positions relative to the N-terminus or the C-terminus of their respective chromosomes.

As used herein, "introgression" refers to the transmission of a desired allele or otherwise selected allele of a genetic locus from one genetic background to another.

As used herein, "primer" refers to an oligonucleotide (synthetic or occurring naturally), which is capable of acting as a point of initiation of nucleic acid synthesis or replication along a complementary strand when placed under conditions in which synthesis of a complementary strand is catalyzed by a polymerase. Typically, primers are about 10 to 30 nucleotides in length, but longer or shorter sequences can be employed. Primers may be provided in double-stranded form, though the single-stranded form is more typically used. A primer can further contain a detectable label, for example a 5' end label.

As used herein, "probe" refers to an oligonucleotide (synthetic or occurring naturally) that is complementary (though not necessarily fully complementary) to a polynucleotide of interest and forms a duplex structure by hybridization with at least one strand of the polynucleotide of interest. Typically, probes are oligonucleotides from 10 to 50 nucleotides in length, but longer or shorter sequences can be employed. A probe can further contain a detectable label.

As used herein, the terms "phenotype," or "phenotypic trait," or "trait" refers to one or more detectable characteristics of a cell or organism which can be influenced by genotype. The phenotype can be observable to the naked eye, or by any other means of evaluation known in the art, e.g., microscopy, biochemical analysis, genomic analysis, an assay for a particular disease tolerance, etc. In some cases, a phenotype is directly controlled by a single gene or genetic locus, e.g., a "single gene trait." In other cases, a phenotype is the result of several genes. In specific embodiments, the phenotype of pea seeds is a yield, height, and/or lodging phenotype.

As used herein, the term "plant" includes plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, pulp, juice, kernels, ears, cobs, husks, stalks, roots, root tips, anthers, and the like. A plant cell is a biological cell of a plant, taken from a plant or derived through culture of a cell taken from a plant. Progeny, variants, and mutants of the regenerated plants are also included within the scope of the invention, provided that these parts comprise the introduced polynucleotides. Further provided is a processed plant product (e.g., extract) or byproduct that retains one or more polynucleotides disclosed herein. A progeny plant can be from any filial generation, e.g., F1, F2, F3, F4, F5, F6, F7, etc. A plant cell is a biological cell of a plant, taken from a plant or derived through culture from a cell taken from a plant.

As used herein, "cross" or "crossing" or "crossed" means to produce progeny via fertilization (e.g. cells, seeds or plants) and includes crosses between plants (sexual) and self-fertilization (selfing). Typically, a cross occurs after pollen is transferred from one flower to another, but those of ordinary skill in the art will understand that plant breeders can leverage their understanding of crossing, pollination, syngamy, and fecundation to circumvent certain steps of the plant life cycle and yet achieve equivalent outcomes, for example, a plant or cell of a pea cultivar described herein. In certain embodiments, a user of this innovation can generate a plant of the claimed invention by removing a genome from its host gamete cell before syngamy and inserting it into the nucleus of another cell. While this variation avoids the unnecessary steps of pollination and syngamy and produces a cell that may not satisfy certain definitions of a zygote, the process falls within the definition of crossing as used herein when performed in conjunction with these teachings. In certain embodiments, the gametes are not different cell types (i.e., egg vs. sperm), but rather the same type and techniques are used to effect the combination of their genomes into a regenerable cell. Other embodiments of crossing include circumstances where the gametes originate from the same parent plant, i.e., a "self" or "self-fertilization." While selfing a plant does not require the transfer of pollen from one plant to another, those of skill in the art will recognize that it nevertheless serves as an example of a cross. Thus, methods and compositions taught herein are not limited to certain techniques or steps that must be performed to create a plant or an offspring plant of the claimed invention, but rather include broadly any method that is substantially the same and/or results in compositions of the claimed invention.

As used herein, a "pea plant" refers to a plant of species *Pisum sativum* L. and includes all plant varieties that can be bred with pea, including wild species such as *Pisum fulvum* and *Pisum sativum* subs *elatius.*

A pea plant or seed with a "high yield" phenotype as used herein refers to a pea plant or pea seed having higher yield than a reference sample of pea plant or seed. In some embodiments a high yield pea plant or seed, or a high yield pea plant or seed population has yield that is at least 10, 11, 12, 13, 14, 15, 16, 17, 18, 19, 20, or more bushels/acre greater than that of a reference pea plant or seed or a reference population of pea plants or seeds. In specific embodiments, a high yield pea plant or seed, or a high yield pea plant or seed population has an average yield of 40, 41, 42, 43, 44, 45, 46, 47, 48, 49, 50, 51, 52, 53, 54, 55, or more bushels/acre. In particular embodiment a high yield pea population comprises an average yield of at least 49, 50, or 51 bushels/acre. In specific embodiments, a high yield pea plant or high yield pea seed or a population of high yield pea plants or seeds has greater yield than a commodity pea seed or commodity pea plant or commodity pea plant or seed population. Commodity peas may have yield of 25, 26, 27, 28, 29, 30, 31, 32, 33, 34, or 35 bushels/acre. In certain embodiments the reference pea plant or seed is a commodity pea plant or commodity pea seed.

A pea plant or seed with a "high height" phenotype as used herein refers to a pea plant (grown from a pea seed) that is taller than a reference sample of pea plant (grown from a reference seed). In some embodiments a high height pea plant (grown from high height pea seed), or a high height pea plant population (grown from a high height pea seed population) is taller by at least 5, 6, 7, 8, 9, 10, 11, 12, 13, 14, 15, or more inches than that of a reference pea plant or a reference population of pea plants. In specific embodiments, a high height pea plant, or a high height pea plant population has an average height of 35, 36, 37, 38, 39, 40, 41, 42, or more inches. In particular embodiment a high height pea population comprises an average height of at least 35-40 inches. In specific embodiments, a high height pea plant, or a high height pea plant population, is taller than a commodity pea plant or a commodity pea plant population. Commodity peas may have height of 20, 21, 22, 23, 24, 25, 26, or 27 inches. In certain embodiments the reference pea plant or seed is a commodity pea plant or commodity pea seed.

A pea plant or seed with a "low lodging" phenotype as used herein refers to a pea plant (grown from a pea seed) having less lodging than a reference sample of pea plant (grown from a reference seed). Lodging as used herein refers to dislocation (e.g., bending or breaking) of plant roots or stems from their vertical stance, and can occur due to external forces (e.g., wind, rain), anatomical reasons (e.g., weak stem structure, tall stem), or heavy fruit/seed load. Lodging can negatively impact crop yield and quality. Lodging can be expressed by lodging units from 1 to 9, 9 being the most severe (i.e., least desirable). In some embodiments a low lodging pea plant or seed, or a low lodging pea plant or seed population has a lodging unit that is at least 1.5, 1.6, 1.7, 1.8, 1.9, 2.0, 2.1, 2.2, 2.3, 2.4, 2.5, or more lodging unit lower than that of a reference pea plant or seed or a reference population of pea plants or seeds. In specific embodiments, a low lodging pea plant or seed, or a low lodging pea plant or seed population has an average lodging unit of 2.8, 2.9, 3.0, 3.1, 3.2, 3.3, or 3.4. In particular embodiment a low lodging pea population comprises an average lodging unit of 3.1 or 3.2. In specific embodiments, a low lodging pea plant, or a low lodging pea plant population, has less severe lodging than a commodity pea plant or a commodity pea plant population. Commodity peas may have a lodging unit of 4.9, 5.0, 5.1, 5.2, 5.3, 5.4, or 5.5. In certain embodiments the reference pea plant or seed is a commodity pea plant or commodity pea seed.

As used herein, a "population of plants," "population of seeds," "plant population," or "seed population" means a set comprising any number, including one, of individuals, objects, or data from which samples are taken for evaluation, e.g., estimating quantitative trait locus (QTL). Most commonly, the terms relate to a breeding population of plants from which members are selected and crossed to produce progeny in a breeding program. A population of plants can include the progeny of a single breeding cross or a plurality of breeding crosses, and can be either actual plants or plant derived material, or *in silico* representations of the plants or seeds. The population members need not be identical to the population members selected for use in subsequent cycles of analyses or those ultimately selected to obtain final progeny plants or seeds. Often, a plant or seed population is derived from a single biparental cross, but may also derive from two or more crosses between the same or different parents. Although a population of plants or seeds may comprise any number of individuals, those of skill in the art will recognize that plant breeders commonly use population sizes ranging from one or two hundred individuals to several thousand, and that the highest performing 5-20% of a population is what is commonly selected to be used in subsequent crosses in order to improve the performance of subsequent generations of the population.

As used herein, the term "crop performance" is used synonymously with "plant performance" and refers to of how well a plant grows under a set of environmental conditions and cultivation practices. Crop performance can be measured by any metric a user associates with a crop's productivity (e.g., yield), appearance and/or robustness (e.g., color, morphology, height, biomass, maturation rate, etc.), product quality (e.g., fiber lint percent, fiber quality, seed protein content, etc.), cost of goods sold (e.g., the cost of creating a seed, plant, or plant product in a commercial, research, or industrial setting) and/or a plant's tolerance to disease (e.g., a response associated with deliberate or spontaneous infection by a pathogen) and/or environmental stress (e.g., drought, flooding, low nitrogen or other soil nutrients, wind, hail, temperature, day length, etc.). Crop performance can also be measured by determining a crop's commercial value and/or by determining the likelihood that a particular inbred, hybrid, or variety will become a commercial product, and/or by determining the likelihood that the offspring of an inbred, hybrid, or variety will become a commercial product. Crop performance can be a quantity (e.g., the volume or weight of seed or other plant product measured in liters or grams) or some other metric assigned to some aspect of a plant that can be represented on a scale (e.g., assigning a 1-10 value to a plant based on its disease tolerance).

A "microbe" will be understood to be a microorganism, i.e. a microscopic organism, which can be single celled or multicellular. Microorganisms are very diverse and include all the bacteria, archaea, protozoa, fungi, and algae, especially cells of plant pathogens and/or plant symbionts. Certain animals are also considered microbes, e.g. rotifers. In various embodiments, a microbe can be any of several different microscopic stages of a plant or animal. Microbes also include viruses, viroids, and prions, especially those which are pathogens or symbionts to crop plants. A "pathogen" as used herein refers to a microbe that causes disease or harmful effects on plant health.

A "fungus" includes any cell or tissue derived from a fungus, for example whole fungus, fungus components, organs, spores, hyphae, mycelium, and/or progeny of the same. A fungus cell is a biological cell of a fungus, taken from a fungus or derived through culture of a cell taken from a fungus.

A "pest" is any organism that can affect the performance of a plant in an undesirable way. Common pests include microbes, animals (e.g. insects and other herbivores), and/or plants (e.g. weeds). Thus, a pesticide is any substance that reduces the survivability and/or reproduction of a pest, e.g. fungicides, bactericides, insecticides, herbicides, and other toxins.

"Tolerance" or "improved tolerance" in a plant to disease conditions (e.g. growing in the presence of a pest) will be understood to mean an indication that the plant is less affected by the presence of pests and/or disease conditions with respect to yield, survivability and/or other relevant agronomic measures, compared to a less tolerant, more "susceptible" plant. Tolerance is a relative term, indicating that a "tolerant" plant survives and/or performs better in the presence of pests and/or disease conditions compared to other (less tolerant) plants (e.g., a different pea cultivar) grown in similar circumstances. As used in the art, "tolerance" is sometimes used interchangeably with "resistance," although resistance is sometimes used to indicate that a plant appears maximally tolerant to, or unaffected by, the presence of disease conditions. Plant breeders of ordinary skill in the art will appreciate that plant tolerance levels vary widely, often representing a spectrum of more-tolerant or less-tolerant phenotypes, and are thus trained to determine the relative tolerance of different plants, plant lines or plant families and recognize the phenotypic gradations of tolerance.

"Yield" as used herein is defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance, photosynthetic carbon assimilation rates, and early vigor may also be important factors in determining yield. Optimizing the abovementioned factors may therefore contribute to increasing crop yield. Yield can be measured and expressed by any means known in the art. In specific embodiments, yield is measured by seed weight or volume in a given harvest area.

As used herein, "selecting" or "selection" in the context of marker-assisted selection or breeding refer to the act of picking or choosing desired individuals, normally from a population, based on certain pre-determined criteria.

As used herein the term "polynucleotide" refers to a single or double stranded nucleic acid sequence which is isolated and provided in the form of an RNA sequence (e.g., an mRNA sequence), a complementary polynucleotide sequence (cDNA), a genomic polynucleotide sequence and/or a composite polynucleotide sequences (e.g., a combination of the above).

The term "isolated" refers to at least partially separated from the natural environment e.g., from a plant cell.

As used herein, the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

In certain embodiments, a user can combine the teachings herein with high-density molecular marker profiles spanning substantially the entire genome of a plant to estimate the value of selecting certain candidates in a breeding program in a process commonly known as genome selection.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable subcombination or as suitable in any other described embodiment of the disclosure. Certain features described in the context of various embodiments are not to be considered essential features of those embodiments, unless the embodiment is inoperative without those elements.

### 2.1 Methods of producing pea plants or seeds with a desirable yield, height, and/or lodging phenotype

In an aspect, this disclosure provides a method of creating a population of pea plants or seeds with a desirable yield, height, and/or lodging phenotype. The method comprises the steps of: (a) genotyping a first population of pea plants or seeds for the presence of at least one yield, height, and/or lodging molecular marker that is within 20 centimorgans of one or more yield, height, and/or lodging Quantitative Trait Locus (QTLs) selected from the group consisting of yield chr2-2 (Ps02_417306163), yield chr5-1 (Ps05_117545329), yield chr5-2 (Ps05_604790207), lodging chr2-1 (Ps02_415727951), lodging chr5-1 (Ps05_608256598), lodging chr6-1 (Ps06_5054240), height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364; (b) selecting from the first population one or more pea plants or seeds comprising one or more yield, height, and/or lodging alleles having the at least one yield, height, and/or lodging molecular marker; and (c) producing a second population of progeny pea plants or seeds from the selected one or more pea plants or plants grown from the selected seeds, wherein the second population of progeny pea plants or seeds comprises the one or more yield, height, and/or lodging alleles having the one or more yield, height, and/or lodging molecular markers, and wherein the second population of progeny pea plants or seeds are pea plants or seeds with a desirable yield, height, and/or lodging phenotype, thereby producing a population of pea plants or seeds with a desirable yield, height, and/or lodging phenotype.

The above-described yield, height, and/or lodging QTLs are further described in Tables 2A, 2B, 6A, 6B, 10A, 10B, and 19-21. Each QTL (such as "Ps01_12111263") comprises the SNP marker associated with a yield, height, and/or lodging phenotype as indicated in Tables 2A, 2B, 6A, 6B, 10A, 10B, and 19-21. The genomic sequence upstream and downstream of the SNP marker of the yield, height, and/or lodging QTL may vary, and can include a nucleic acid sequence that has at least 85% sequence identity with the nucleic acid sequence described in Tables 2A, 2B, 6A, 6B, 10A, 10B, and 19-21. Each marker listed in Tables Tables 2A, 2B, 6A, 6B, 10A, 10B, and 19-21 (referred to as "marker AA") refers to an SNP marker at position 101 of the nucleic acid sequence identified for the marker AA (SEQ ID NO: BB) in a genomic region comprising the nucleic acid sequence of SEQ ID NO: BB, or at a corresponding position of a genomic region at least about 50, 100, 150, or 200 nucleotides of which is aligned to SEQ ID NO: BB for maximum homology (e.g., 85%, 86%, 87%, 88%, 89%, 90%, 91%, 92%, 93%, 94%, 95%, 96%, 97%, 98%, or 99% homology) when using standard alignment parameters. For example, the QTL identified as Ps01_12111263 can have a sequence that has at least 85% identity with SEQ ID NO: 15. The marker identified as Ps01_12111263 can also refer to an SNP marker at position 101 of a nucleic acid sequence of SEQ ID NO: 15 in a genomic region comprising the nucleic acid sequence of SEQ ID NO: 15, or at a corresponding position of a genomic region at least about 50, 100, 150, or 200 nucleotides which is aligned to SEQ ID NO: 15 for maximum homology (e.g., 85%, 86%, 87%, 88%, 89%, 90%, 91%, 92%, 93%, 94%, 95%, 96%, 97%, 98%, or 99% homology) when using standard alignment parameters.

Yield, height, and/or lodging QTLs can be one or more of Ps02_406013484, Ps02_415945622, yield chr2-2 (Ps02_417306163), height chr2-2 (Ps2_390480167), lodging chr2-1 (Ps02_415727951), Ps06_32259152, Ps06_ 43753843, height chr6-1 (Ps06_38889673), Ps07_129103458, height chr7-2 (Ps07_129458029), Ps03_71303964, height chr3-1 (Ps03_74158043), Ps05_583707944, Ps05_612824899, Ps05_616716582, lodging chr5-1 (Ps05_608256598), yield chr5-2 (Ps05_604790207), Ps06_12615016, Ps06_33377536, height chr6-1 (Ps06_38889673), Ps07_133823647, Ps07_112377551, height chr7-1 (Ps07_115631962), Ps04_205866569, height chr4-1 (Ps04_192115819), Ps07_114916793, Ps07_234681662, Ps07_238767894, and height chr7-3 (Ps07_256289873). In specific embodiments, yield, height, and/or lodging QTLs are Ps02_406013484, Ps02_415945622, yield chr2-2 (Ps02_417306163), height chr2-2 (Ps2_390480167), lodging chr2-1 (Ps02_415727951), Ps05_583707944, Ps05_612824899, lodging chr5-1 (Ps05_608256598), yield chr5-2 (Ps05_604790207), Ps07_112377551, height chr7-1 (Ps07_115631962), Ps04_205866569, height chr4-1 (Ps04_192115819), Ps07_114916793, Ps07_234681662, Ps07_238767894, and height chr7-3 (Ps07_256289873).

Yield, height, and lodging QTLs can coincide. Accordingly, yield, height, and/or lodging QTLs can be pleiotropic QTLs, i.e., QTLs that are associated with more than one of yield, height, and lodging phenotypes. For example, yield, height, and/or lodging QTLs can be one or more of Ps02_406013484, Ps03_531239107, Ps04_445153308, and Ps07_9801763.

Yield QTLs, i.e., QTLs associated with a yield phenotype, can be one or more of chr2-2 (Ps02_417306163), yield chr5-1 (Ps05_117545329), yield chr5-2 (Ps05_604790207), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, and Ps07_481516842.

Height QTLs, i.e., QTLs associated with a height phenotype, can be one or more of height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps03_531239107, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364.

Lodging QTLs, QTLs associated with a lodging phenotype, can be one or more of lodging chr2-1 (Ps02_415727951), lodging chr5-1 (Ps05_608256598), lodging chr6-1 (Ps06_5054240), Ps02_406013484, Ps04_445153308, Ps07_9801763, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515.

In some embodiments, at least one yield, height, and/or lodging molecular marker is within 0.5, 1, 1.5, 2, 2.5,, 3, 3.5, 4, 4.5, 5, 5.5, 6. 6.5, 7, 7.5, 8, 8.5, 9, 9.5, or 10 centimorgans of said one or more yield, height, and/or lodging QTLs.

In one embodiment of the method, the yield, height, and/or lodging QTL is Ps03_531239107 and/or Ps05_49389403. In another embodiment, the yield, height, and/or lodging QTL is Ps03_531014546, Ps03_531232613, and/or Ps03_531239107.

In some embodiments, selecting from the first population one or more pea plants or seeds is based on detection of the presence of a yield, height, and/or lodging haplotype. A yield, height, and/or lodging haplotype can comprise yield, height, and/or lodging alleles of two or more polymorphic loci described herein.

Provided herein are methods of producing a population of pea plants or seeds with a desirable yield, height, and/or lodging phenotype. Methods of producing a population of pea plants or seeds with a desirable yield, height, and/or lodging phenotype combining commercially significant yield and a desirable yield, height, and/or lodging phenotype without a corresponding reduction in protein or oil content are disclosed herein. In some embodiments, methods of producing a pea plant or seed or a population of pea plants or seeds with a desirable yield, height, and/or lodging phenotype with yield that is at least 10, 11, 12, 13, 14, 15, 16, 17, 18, 19, 20, or more bushels/acre greater than that of a reference pea plant or seed or a reference population of pea plants or seeds are provided. The effect size of yield QTLs provided herein can be 10-20 bushels/acre, such as 11-20, 12-20, 13-20, 13-19, 12-19, or 11-19 bushels/acre. Methods provided herein can produce a high yield pea plant or seed, or a high yield pea plant or seed population that has an average yield of 40, 41, 42, 43, 44, 45, 46, 47, 48, 49, 50, 51, 52, 53, 54, 55, or more bushels/acre. In particular embodiment the pea plant or seed population provided herein comprises an average yield of at least 49, 50, or 51 bushels/acre. A pea plant or pea seed produced by the methods provided herein can have greater yield than a commodity pea seed or commodity pea plant. Commodity peas may have yield of 25, 26, 27, 28, 29, 30, 31, 32, 33, 34, or 35 bushels/acre. The reference pea plant or seed can be a commodity pea plant or commodity pea seed, a pea plant or seed that has not been bred using the methods provided herein, or a pea plant or seed that does not contain the yield, height, and/or lodging QTLs provided herein.

In some embodiments, methods of producing a pea plant or a population of pea plants with a desirable yield, height, and/or lodging phenotype with height that is taller by at least 5, 6, 7, 8, 9, 10, 11, 12, 13, 14, 15, or more inches than that of a reference pea plant or a reference population of pea plants are provided. The effect size of height QTLs provided herein can be 5-15 inches, such as 6-15, 7-15, 8-15, 9-15, 10-15, 8-14, 9-14, or 10-14 inches. Methods provided herein can produce a high height pea plant, or a high height pea plant population that has an average height of 35, 36, 37, 38, 39, 40, 41, 42, or more inches. In particular embodiment the pea population provided herein comprises an average height of at least 35-40 inches. A pea plant produced by the methods provided herein can be taller than a commodity pea plant. Commodity peas may have height of 20, 21, 22, 23, 24, 25, 26, or 27 inches. The reference pea plant or seed can be a commodity pea plant or commodity pea seed, a pea plant or seed that has not been bred using the methods provided herein, or a pea plant or seed that does not contain the yield, height, and/or lodging QTLs provided herein.

In some embodiments, methods of producing a pea plant or a population of pea plants with a desirable yield, height, and/or lodging phenotype having less lodging by at least 1.5, 1.6, 1.7, 1.8, 1.9, 2.0, 2.1, 2.2, 2.3, 2.4, 2.5, or more lodging unit than that of a reference pea plant or seed or a reference population of pea plants or seeds are provided. Lodging can be expressed on the scale of 1-9 lodging units, with 9 being the least desirable (high lodging and easy to fall). The effect size of lodging QTLs provided herein can be 1.5-2.3 lodging units, such as 1.5-2.0, 1.6-2.3, 1.6-2.2, 1.6-2.1, 1.6-2.0. 1.7-2.3, 1.7-2.2, 1.7-2.0, or 1.8-2.3 lodging units. Methods provided herein can produce a low lodging pea plant, or a low lodging pea plant population that has an average lodging unit of 2.8, 2.9, 3.0, 3.1, 3.2, 3.3, or 3.4. In particular embodiment the pea population provided herein comprises an average lodging unit of 3.1 or 3.2. A pea plant produced by the methods provided herein can have less lodging than a commodity pea plant. Commodity peas may have a lodging unit of 4.9, 5.0, 5.1, 5.2, 5.3, 5.4, or 5.5. The reference pea plant or seed can be a commodity pea plant or commodity pea seed, a pea plant or seed that has not been bred using the methods provided herein, or a pea plant or seed that does not contain the yield, height, and/or lodging QTLs provided herein.

In specific embodiments, the population of pea plants or seeds having one or more yield, height, and/or lodging alleles produced by the methods provided herein have average yield that is greater by at least 13 bushels per acre, average height that is greater by at least 8 inches, and/or average lodging that is lower by at least 1.5 lodging units relative to pea plants or seeds without said one or more yield, height, and/or lodging alleles.

In specific embodiments, the population of pea plants or seeds with a desirable yield, height, and/or lodging phenotype have average protein or average oil content that is not significantly lower than a control population. For example, the population of pea plants or seeds having one or more yield, height, and/or lodging alleles can have average protein or average oil content that is at least 90%, 91%, 92%, 93%, 94%, 95%, 96%, 97%, 98%, 99%, 100%, or more relative to a control population of pea plants or seeds without said one or more yield, height, and/or lodging alleles.

QTLs (i.e., yield, height, and/or lodging QTLs) that exhibit significant co-segregation with desirable yield, height, and/or lodging phenotype are provided herein. In specific embodiments, plants or seeds comprising the yield, height, and/or lodging QTLs further comprise one or more allele associated with high protein or oil content. In some embodiments, the one or more allele associated with high protein or oil is within 10 centimorgans or less, e.g., 9.5 centimorgans or less, 9 centimorgans or less, 8.5 centimorgans or less, 8 centimorgans or less, 7.5 centimorgans or less, 7 centimorgans or less, 6.5 centimorgans or less, 6 centimorgans or less, 5.5 centimorgans or less, 5 centimorgans or less, 4.5 centimorgans or less, 4 centimorgans or less, 3.5 centimorgans or less, 3 centimorgans or less, 2.5 centimorgans or less, 2 centimorgans or less, 1.5 centimorgans or less, 1 centimorgans or less, or 0.5 centimorgans or less from one or more yield, height, and/or lodging QTLs. Yield, height, and/or lodging QTLs can be tracked during plant breeding or introgressed into a desired genetic background in order to provide plants exhibiting a desirable yield, height, and/or lodging phenotype and, in specific embodiments, one or more other beneficial traits. In an aspect, this disclosure identifies QTL intervals that are associated with a desirable yield, height, and/or lodging phenotype in different pea varieties described herein.

In specific embodiments, yield, height, and/or lodging molecular markers are associated with a plants or plant parts having higher yield, higher height, and/or less lodging than corresponding plants or plant parts without the yield, height, and/or lodging molecular marker. The yield in plants and plant parts having at least one yield, height, and/or lodging molecular marker (e.g., SNP or deletion marker) disclosed herein can be greater by at least about 5%, 10%, 15%, 20%, 25%, 30%, 35%, 40%, 45%, 50%, 55%, 60%, 65%, 70%, 75%, 80%, 85%, or more relative to corresponding plants or plant parts without the yield, height, and/or lodging molecular marker. The height in plants and plant parts having at least one yield, height, and/or lodging molecular marker (e.g., SNP or deletion marker) disclosed herein can be greater by at least about 5%, 10%, 15%, 20%, 25%, 30%, 35%, 40%, 45%, 50%, 55%, 60%, 65%, 70%, 75%, 80%, 85%, or more relative to corresponding plants or plant parts without the yield, height, and/or lodging molecular marker. The lodging in plants and plant parts having at least one yield, height, and/or lodging molecular marker (e.g., SNP or deletion marker) disclosed herein can be decreased by at least about 5%, 10%, 15%, 20%, 25%, 30%, 35%, 40%, 45%, 50%, 55%, 60%, 65%, 70%, 75%, 80%, 85%, or more relative to corresponding plants or plant parts without the yield, height, and/or lodging molecular marker.

Yield, height, and/or lodging markers of the present disclosure include "dominant" or "codominant" markers. "Codominant markers" reveal the presence of two or more alleles (two per diploid individual). "Dominant markers" reveal the presence of only a single allele. The presence of the dominant marker phenotype (e.g., a band of DNA) is an indication that one allele is present in either the homozygous or heterozygous condition. The absence of the dominant marker phenotype (e.g., absence of a DNA band) is merely evidence that "some other" undefined allele is present. In the case of populations where individuals are predominantly homozygous and loci are predominantly dimorphic, dominant and codominant markers can be equally valuable. As populations become more heterozygous and multiallelic, codominant markers often become more informative of the genotype than dominant markers.

Yield, height, and/or lodging markers, such as simple sequence repeat markers (SSR), AFLP markers, RFLP markers, RAPD markers, phenotypic markers, single nucleotide polymorphisms (SNPs), isozyme markers, deletion markers, microarray transcription profiles that are genetically linked to or correlated with alleles of a QTL of the present invention can be utilized (Walton, Seed World 22-29 (July, 1993), Burow et al., Molecular Dissection of Complex Traits, 13-29, ed. Paterson, CRC Press, New York (1988)). Methods to isolate and identify such markers are known in the art. For example, locus-specific SSR markers can be obtained by screening a genomic library for microsatellite repeats, sequencing of "positive" clones, designing primers which flank the repeats, and amplifying genomic DNA with these primers. The size of the resulting amplification products can vary by integral numbers of the basic repeat unit. To detect a polymorphism, PCR products can be radiolabeled, separated on denaturing polyacrylamide gels, and detected by autoradiography. Fragments with size differences > 4 bp can also be resolved on agarose gels, thus avoiding radioactivity.

SNPs occur at a single nucleotide. SNPs are more stable than other classes of polymorphisms. Their spontaneous mutation rate is approximately 10-9 (Kornberg, DNA Replication, W. H. Freeman & Co., San Francisco (1980)). As SNPs result from sequence variation, new polymorphisms can be identified by sequencing random genomic or cDNA molecules. SNPs can also result from deletions, point mutations and insertions. That said, SNPs are also advantageous as markers since they are often diagnostic of "identity by descent" because they rarely arise from independent origins. Any single base alteration, whatever the cause, can be an SNP. SNPs occur at a greater frequency than other classes of polymorphisms and can be more readily identified. In the present disclosure, an SNP can represent a single indel event, which may consist of one or more base pairs, or a single nucleotide polymorphism.

A marker (e.g., an SNP marker) associated with a phenotype (e.g., yield, height, lodging) can be a positive marker or a negative marker. A "positive marker" as used herein refers to a marker in which the allele has a positive effect on the phenotype (e.g., high yield, high height, high lodging). A "negative marker" as used herein refers to a marker in which the allele has a negative effect on the phenotype (e.g., low yield, low height, low lodging). A positive marker can be associated with a desirable or undesirable phenotype. For example, a positive yield marker (associated with high yield) is usually considered to be associated with a desirable phenotype, whereas a positive lodging marker (associated with high lodging) is usually considered to be associated with an undesirable phenotype. A positive height marker (associated with high height) can be associated with higher yield, but can also be associated with susceptibility to lodging. Thus, a positive height marker is considered to be associated with an undesirable phenotype (e.g., high lodging, tendency to lodge) or a desirable phenotype (e.g., high yield) depending on other phenotypes and circumstances. For example, breeders may select for shorter plants as desirable over taller plants when the yield is similar.

As used herein, a "reference allele" refers to one variation of the SNP sequence (e.g., a nucleotide), and an "variant allele" refers to another variation of the SNP sequence (e.g., a nucleotide). Alleles can also be referred to as a "major allele" (referring to the most common (or frequent) variation of a sequence (e.g., a nucleotide)), and a "minor allele" (referring to a less common (or frequent) variation of a sequence (e.g., a nucleotide). Example reference and variant alleles for yield, height, and/or lodging markers are set forth for instance in Tables 2A, 2B, 6A, 6B, 10A, 10B, and 19-21. Tables 19-21 set forth example yield, height, and/or lodging markers with marker weight, as expressed by least absolute shrinkage and selection operator (LASSO) protein coefficient. A "marker weight" as used herein, expressed in some embodiments as a LASSO coefficient (e.g., a LASSO yield, height, and/or lodging coefficient), refers to the significance of association of the marker with a positive or negative yield, height, and/or lodging phenotype. A positive LASSO coefficient (positive marker weight) shown in Tables 19-21 indicates that the variant allele has a positive effect on the phenotype (e.g., the variant allele is associated with high yield, high height, and/or high lodging), where the variant allele is a positive marker and the reference allele is a negative marker. A negative LASSO coefficient (negative marker weight) shown in Tables 19-21 indicates that the variant allele has a negative effect on the phenotype (e.g., the variant allele is associated with low yield, low height, and/or low lodging), where the variant allele is a negative marker and the reference allele is a positive marker. In some embodiments, a marker weight greater than the upper cut-off value or less than the lower cut-off value indicates a significant association of the marker with a yield, height, and/or lodging phenotype. The cut-off value can be determined by one skilled in the art. For example, a marker weight greater than 0.01 or less than 0.01; greater than 0.02 or less than 0.02; greater than 0.025 or less than 0.025; greater than 0.03 or less than 0.03; greater than 0.04 or less than 0.04; greater than 0.05 or less than 0.05; or greater than 0.1 or less than 0.1. For example, in Table 19, the SNP marker Ps02_406013484 has a positive yield LASSO coefficient and a negative lodging LASSO coefficient. Thus, the variant allele C is associated with a desirable phenotype of high yield and low lodging. The SNP marker Ps04_445153308 has a negative LASSO coefficient and a positive LASSO coefficient. Thus, the reference allele C is associated with a desirable phenotype of high yield and low lodging.

An "anchor marker" as used herein refers to an SNP marker that has a significant association with a desirable yield, height, and/or lodging phenotype, and includes a positive marker and a negative marker. Each anchor marker can have one or more neighboring markers (SNP markers), also referred to as "satellite" markers (SNP markers). The distance between the anchor marker and the satellite marker can be any distance, for example 0.001 centimorgan to 10 centimorgan, e.g., about 0.001-0.01, 0.01-1, or 1-10 centimorgan. One or more satellite markers can be used to increase the distance (e.g., centimorgan) from the anchor marker within which the anchor marker can exert its association with desirable yield, height, and/or lodging phenotype, or can accurately predict a yield, height, and/or lodging plant. For example, the methods of producing a population of pea plants or seeds with a desirable yield, height, and/or lodging phenotype provided herein can comprise genotyping a first population of pea plants or seeds for the presence of at least one yield, height, and/or lodging anchor marker that is within a certain distance from the yield, height, and/or lodging QTL, e.g., 10 centimorgans (e.g., 0.5, 1, 1.5, 2, 2.5, 3, 3.5, 4, 4.5, 5, 5.5, 6, 6.5, 7, 7.5, 8, 8.5, 9, 9.5, 10) from the yield, height, and/or lodging QTL, or the presence of at least one satellite marker associated with the anchor marker that is within a longer distance from the yield, height, and/or lodging QTL, e.g., 20 centimorgans (e.g., 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20) from the yield, height, and/or lodging QTL. Similarly, the methods of introgressing a yield, height, and/or lodging QTL provided herein can comprise selecting a progeny plant or seed comprising a yield, height, and/or lodging allele of a polymorphic locus linked to the yield, height, and/or lodging QTL, wherein the polymorphic locus can be an anchor marker that is within a certain distance from the yield, height, and/or lodging QTL, e.g., 10 centimorgans (e.g., 0.5, 1, 1.5, 2, 2.5, 3, 3.5, 4, 4.5, 5, 5.5, 6, 6.5, 7, 7.5, 8, 8.5, 9, 9.5, 10) from the yield, height, and/or lodging QTL, or the polymorphic locus can be a satellite marker associated with the anchor marker that is within a longer distance from the yield, height, and/or lodging QTL, e.g., 20 centimorgans (e.g., 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20) from the yield, height, and/or lodging QTL.

In some embodiments an SNP marker at yield, height, and/or lodging QTL comprises the SNP at the positions described in Tables 2A, 2B, 6A, 6B, 10A, 10B, and 19-21. For example, an SNP marker at yield, height, and/or lodging QTL associated with a desirable yield, height, and/or lodging phenotype can contain T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A or G at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; G at position 195183596 of chromosome 7LG7; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of a *Pisum sativum* genome. In specific embodiments, the *Pisum sativum* genome referred to herein is the Cameor v1a reference genome, or the positions on chromosomes provided herein correspond to respective positions (the same numbered positions) of the *Pisum sativum* Cameor vla reference genome.

In specific embodiment, the yield, height, and/or lodging QTL comprises a deletion marker. As used herein, a "deletion marker" refers to a deletion of a nucleotide region in the genome of plants or plant parts exhibiting a yield, height, and/or lodging phenotype. Plants or plant parts having genomes lacking the deletion marker exhibit a lower protein content by weight than the plants and plant parts having genomes with the deletion marker. The deleted nucleotide region of a deletion marker can be a deletion of any number of consecutive nucleotides that is associated with a yield, height, and/or lodging phenotype. For example, the deletion can be 2-500 bp, 5-250 bp, 10-200 bp, 20-180 bp, 40-160bp, 50-140bp, 60-120bp, 70-100 bp, 80-100 bp, 85-95 bp, or about 2 bp, 5 bp, 10 bp, 15 bp, 20 bp, 25 bp, 30 bp, 35 bp, 40 bp, 45 bp, 50 bp, 55 bp, 60 bp , 65 bp, 70 bp, 75 bp, 80 bp, 81 bp, 82 bp, 83 bp, 84 bp, 85 bp, 86 bp, 87 bp, 88 bp, 89 bp, 90 bp, 91 bp, 92 bp, 93 bp, 94 bp, 95 bp, 96 bp, 97 bp, 100 bp, 105 bp, 110 bp, 120 bp, 130 bp, 140 bp, 150 bp, 160 bp, 170 bp, 180 bp, 200 bp, 225 bp, 250 bp, 275 bp, 300 bp, 350 bp, 400 bp, 450 bp, or about 500 bp. In certain embodiments, the deletion marker is 87 bp, 88 bp, or 89 bp.

In specific embodiments, the deletion maker can be wholly or at least partially within a gene. The deletion marker can be wholly or at least partially within an exon or intron of the gene. That is, the deletion marker can be a deletion of a nucleotide sequence entirely within a gene or spanning the 5' end of the gene or the 3' of the gene. In some embodiments, the deletion marker eliminates the start codon of a gene. The deletion marker can also account for removal of a signal peptide of a gene. In some embodiments, the deletion marker eliminates both the start codon and the signal peptide of a gene. The gene can be any gene in the genome.

The yield, height, and/or lodging QTLs disclosed herein can be an expression QTL (eQTL). As used herein an eQTL refers to a QTL that is associated with differential expression of a gene. In specific embodiments, when a QTL is present in the genome, a gene associated with the eQTL is has reduced expression. For example, the presence of an eQTL can eliminate or substantially elimination expression of a gene.

As disclosed herein, a pea plant or seed refers to a plant, plant part, or seed of *Pisum sativum.* In specific embodiments, all chromosomal positions listed herein are identified relative to the reference genome, such as the Cameor vla reference genome. The wild perennial peas belong to the genus *Pisum* and have a wide array of genetic diversity. In some embodiments described herein, the pea plant or seed is a members of the genus *Pisum,* such as *Pisum sativum* and *Pisum fulvum.*

In specific embodiments, the plants or plant parts with a desirable yield, height, and/or lodging phenotype, or plant products produced from the plants or plant parts with a desirable yield, height, and/or lodging phenotype comprise at least one yield, height, and/or lodging QTL disclosed herein. For example, in specific embodiments, a pea seed or pea protein product (e.g., pea protein concentrate, pea protein, or pea protein isolate) contains at least one marker selected from the group consisting of yield chr2-2 (Ps02_417306163), yield chr5-1 (Ps05_117545329), yield chr5-2 (Ps05_604790207), lodging chr2-1 (Ps02_ 415727951), lodging chr5-1 (Ps05_ 608256598), lodging chr6-1 (Ps06_ 5054240), height chr2-2 (Ps2_390480167), height chr3-1 (Ps03_ 74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03 253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364.

### 2.2 Methods of introgressing a vield, height, and/or lodging QTL

Provided herein are methods for selection and introgression of a yield, height, and/or lodging QTL. The methods comprise the steps of (a) crossing a first pea plant comprising a yield, height, and/or lodging QTL with a second pea plant of a different genotype to produce one or more progeny plants or seeds; and (b) selecting a progeny plant or seed comprising a yield, height, and/or lodging allele of a polymorphic locus linked to the yield, height, and/or lodging QTL. The polymorphic locus described herein corresponds to a chromosomal segment comprising any marker within the genomic region between positions: 386683682-424059129 of chromosome 2; 57407964-130197960 of chromosome 5, 541653217-end of chromosome 5, 387250594-427410060 of chromosome 2; 547677746-end of chromosome 5; start-17888751 of chromosome 6; 354324649-402078389 of chromosome 2; 18873917-69320372 of chromosome 3; 273327332-422042733 of chromosome 3; 118944413-200876233 of chromosome 4; 17641704-56905305 of chromosome 6; 79787890-153689044 of chromosome 7; 91738904-165762735 of chromosome 7; 207998578-360550624 of chromosome 7; scaffold00011; 73778015-347862320 of chromosome 1 LG6; 287196406-405820442 of chromosome 2 LG1; scaffold05541; 22316149-374998060 of chromosome 4 LG4; 35018291-496475925 of chromosome 5 LG3; 4102 if scaffold00839; 332130985-426328693 of chromosome 6 LG2; 8316115-481251260 of chromosome 7 LG7; scaffold02229; 122338217-340372022 of chromosome 1 LG6; 12117951-41766109 of chromosome 2 LG1; 55052537-420398638 of chromosome 3 LG5; scaffold06018; scaffold03592; super-scaffold8606; 47145729-440853776 of chromosome 4 LG4; scaffold02661; 3579419-570360377 of chromosome 5LG3; super-scaffold9678; 2560210-348314640 of chromosome 6 LG2; 191955823-63397608 of chromosome 7 LG7; 63453702 of chromosome 1 LG6; 96628783-415226641 of chromosome 2 LG1; scaffold05147; 403366757 of chromosome 4 LG4; scaffold00254; 811842-294741093 of chromosome 3 LG5; scaffold03249; 173468835-440508849 of chromosome 4 LG4; 362378-229071963 of chromosome 5 LG3; scaffold01526; 157000374-469077464 of chromosome 6 LG2; scaffold01649; or 750954-487220871 of chromosome 7 LG7 of a *Pisum sativum* genome. The genomic region described herein can correspond to a respective genomic region, i.e., a genomic region between positions: 386683682-424059129 of chromosome 2; 57407964-130197960 of chromosome 5; 541653217-end of chromosome 5; 387250594-427410060 of chromosome 2; 547677746-end of chromosome 5; start-17888751 of chromosome 6; 354324649-402078389 of chromosome 2; 18873917-69320372 of chromosome 3; 273327332-422042733 of chromosome 3; 118944413-200876233 of chromosome 4; 17641704-56905305 of chromosome 6; 79787890-153689044 of chromosome 7; 91738904-165762735 of chromosome 7; 207998578-360550624 of chromosome 7; scaffold00011; 73778015-347862320 of chromosome 1 LG6; 287196406-405820442 of chromosome 2 LG1; scaffold05541; 22316149-374998060 of chromosome 4 LG4; 35018291-496475925 of chromosome 5 LG3; 4102 if scaffold00839; 332130985-426328693 of chromosome 6 LG2; 8316115-481251260 of chromosome 7 LG7; scaffold02229; 122338217-340372022 of chromosome 1 LG6; 12117951-41766109 of chromosome 2 LG1; 55052537-420398638 of chromosome 3 LG5; scaffold06018; scaffold03592; super-scaffold8606; 47145729-440853776 of chromosome 4 LG4; scaffold02661; 3579419-570360377 of chromosome 5LG3; super-scaffold9678; 2560210-348314640 of chromosome 6 LG2; 191955823-63397608 of chromosome 7 LG7; 63453702 of chromosome 1 LG6; 96628783-415226641 of chromosome 2 LG1; scaffold05147; 403366757 of chromosome 4 LG4; scaffold00254; 811842-294741093 of chromosome 3 LG5; scaffold03249; 173468835-440508849 of chromosome 4 LG4; 362378-229071963 of chromosome 5 LG3; scaffold01526; 157000374-469077464 of chromosome 6 LG2; scaffold01649; or 750954-487220871 of chromosome 7 LG7 of the *Pisum sativum* Cameor vla reference genome.

Selecting the progeny plant or seed from the population can be based on the presence of a yield, height, and/or lodging haplotype. In particular embodiments, a yield, height, and/or lodging haplotype comprises alleles of two or more polymorphic loci described herein.

The yield QTL can contain an SNP marker associated with high yield; the height QTL can contain an SNP marker associated with height; and the lodging QTL can contain an SNP marker associated with low lodging.

In the method of introgressing a yield, height, and/or lodging QTL, the yield, height, and/or lodging SNP (e.g., a desirable SNP) can be one or more of: yield chr2-2 (Ps02_417306163), yield chr5-1 (Ps05_117545329), yield chr5-2 (Ps05_604790207), lodging chr2-1 (Ps02_ 415727951), lodging chr5-1 (Ps05_ 608256598), lodging chr6-1 (Ps06_ 5054240), height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02 389051201, Ps02 406013484, Ps02 415945622, Ps03 98286373, Ps04 33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03 205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364. In some embodiments, the SNP marker is selected from the group consisting of: T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A or G at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; G at position 195183596 of chromosome 7LG7; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of a *Pisum sativum* genome. The *Pisum sativum* genome referred to herein can be the Cameor v1a reference genome, or the positions on chromosomes provided herein correspond to respective positions (the same numbered positions) of the *Pisum sativum* Cameor v1a reference genome.

In some embodiments, the SNP marker is selected from the group consisting of Ps02_406013484, Ps02_415945622, yield chr2-2 (Ps02_417306163), height chr2-2 (Ps2_390480167), lodging chr2-1 (Ps02_ 415727951), Ps06_32259152, Ps06_43753843, height chr6-1 (Ps06_38889673), Ps07_129103458, height chr7-2 (Ps07_129458029), Ps03_71303964, height chr3-1 (Ps03_ 74158043), Ps05_583707944, Ps05_612824899, Ps05_616716582, lodging chr5-1 (Ps05_ 608256598), yield chr5-2 (Ps05_604790207), Ps06_12615016, Ps06_33377536, height chr6-1 (Ps06_38889673), Ps07_133823647, Ps07_112377551, height chr7-1 (Ps07_115631962), Ps04_205866569, height chr4-1 (Ps04_192115819), Ps07_114916793, Ps07_234681662, Ps07_238767894, and height chr7-3 (Ps07_256289873). The SNP marker can be one or more of: C at position 397000005 of chromosome 2LG1; G at position 405820442 of chromosome 2LG1; T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T or C at position 31021563 of chromosome 6LG2; A at position 132484304 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; T at position 543282804 of chromosome 5LG3; T at position 570360377 of chromosome 5LG3; C at position 8937964 of chromosome 5LG3; G at position 566655216 of chromosome 5; C at position 563627520 of chromosome 5; A at position 11907302 of chromosome 6LG2; A at position 2560210 of chromosome 6LG2; T or C at position 31021563 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A or G at position 45871371 of chromosome 7LG7; A or G at position 115361106 of chromosome 7LG7; T or G at position 173468835 of chromosome 4LG4; T or C at position 158451622 of chromosome 4; T at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; A or G at position 116467927 of chromosome 7LG7; C or T at position 293412012 of chromosome 7LG7; T or G at position 310516174 of chromosome 7LG7; A or G at position 324031049 of chromosome 7; A or G at position 13857 of scaffold 04080; and A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity of the *Pisum sativum* genome. The *Pisum sativum* genome referred to herein can be the Cameor v1a reference genome, or the positions on chromosomes provided herein correspond to respective positions (the same numbered positions) of the *Pisum sativum* Cameor v1a reference genome. The foregoing markers have been identified in the overlapping chromosome regions in the GWAS population and in the LASSO model.

In some embodiments, the SNP marker is selected from the group consisting of Ps02_406013484, Ps02_415945622, yield chr2-2 (Ps02_417306163), height chr2-2 (Ps2_390480167), lodging chr2-1 (Ps02_ 415727951), Ps05_583707944, Ps05_612824899, lodging chr5-1 (Ps05_ 608256598), yield chr5-2 (Ps05_604790207), Ps07_112377551, height chr7-1 (Ps07_115631962), Ps04_205866569, height chr4-1 (Ps04_192115819), Ps07_114916793, Ps07_234681662, Ps07_238767894, and height chr7-3 (Ps07_256289873). The SNP marker can be one or more of: C at position 397000005 of chromosome 2LG1; G at position 405820442 of chromosome 2LG1; T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; T at position 543282804 of chromosome 5LG3; T at position 570360377 of chromosome 5LG3; G at position 566655216 of chromosome 5; C at position 563627520 of chromosome 5; A or G at position 45871371 of chromosome 7LG7; A or G at position 115361106 of chromosome 7LG7; T or G at position 173468835 of chromosome 4LG4; T or C at position 158451622 of chromosome 4; T at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; A or G at position 116467927 of chromosome 7LG7; C or T at position 293412012 of chromosome 7LG7; T or G at position 310516174 of chromosome 7LG7; A or G at position 324031049 of chromosome 7; A or G at position 13857 of scaffold 04080; and A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity of the *Pisum sativum* genome. The *Pisum sativum* genome referred to herein can be the Cameor v1a reference genome, or the positions on chromosomes provided herein correspond to respective positions (the same numbered positions) of the *Pisum sativum* Cameor v1a reference genome. The foregoing markers have been identified in the overlapping chromosome regions in the GWAS population and in the LASSO model, with overlapping markers shown to associate with the same phenotype.

In some embodiments, the SNP marker is a pleiotropic marker and is selected from the group consisting of Ps02_406013484, Ps03_531239107, Ps04_445153308, and Ps07_9801763. The SNP marker can be one or more of: C at position 397000005 of chromosome 2LG1; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; C at position 374998060 of chromosome 4LG4; and A at position 8316115 of chromosome 7LG7 of the *Pisum sativum* genome. The *Pisum sativum* genome referred to herein can be the Cameor v1a reference genome, or the positions on chromosomes provided herein correspond to respective positions (the same numbered positions) of the *Pisum sativum* Cameor v1a reference genome. The foregoing markers have identified as pleiotropic markers, i.e., being associated with more than one phenotypes (i.e., two or more of yield, height, and lodging).

The SNP marker associated with high yield can be one or more of yield chr2-2 (Ps02_417306163), yield chr5-1 (Ps05_117545329), yield chr5-2 (Ps05_604790207), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, and Ps07_481516842. The yield SNP marker can be one or more of: T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; and T at position 481251260 of chromosome 7LG7 of the *Pisum sativum* genome. The *Pisum sativum* genome referred to herein can be the Cameor v1a reference genome, or the positions on chromosomes provided herein correspond to respective positions (the same numbered positions) of the *Pisum sativum* Cameor v1a reference genome.

The SNP marker associated with height can be one or more of height chr2-2 (Ps2_390480167), height chr3-1 (Ps03_ 74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps03_531239107, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364. The height SNP marker can be selected from the group consisting of: C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A or G at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of the *Pisum sativum* genome. The *Pisum sativum* genome referred to herein can be the Cameor v1a reference genome, or the positions on chromosomes provided herein correspond to respective positions (the same numbered positions) of the *Pisum sativum* Cameor v1a reference genome.

The SNP marker associated with low lodging can be one or more of lodging chr2-1 (Ps02_ 415727951), lodging chr5-1 (Ps05_ 608256598), lodging chr6-1 (Ps06_ 5054240), Ps02_406013484, Ps04_445153308, Ps07_9801763, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, and Ps07_195412515. The lodging SNP marker can be one or more of: C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C at position 397000005 of chromosome 2LG1; C at position 374998060 of chromosome 4LG4; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; and G at position 195183596 of chromosome 7LG7 of the *Pisum sativum* genome. The *Pisum sativum* genome referred to herein can be the Cameor v1a reference genome, or the positions on chromosomes provided herein correspond to respective positions (the same numbered positions) of the *Pisum sativum* Cameor v1a reference genome.

In further embodiment, the present disclosure provides methods for introgressing multiple yield, height, and/or lodging QTLs identified herein to generate a population of pea plants or seeds with a high yield, optimal height, and/or low lodging phenotype. The yield, height, and/or lodging QTLs can be selected from, and any combination of, those provided herein.

In some embodiments, provided herein are methods for concurrently introgressing at least one or more, two or more, three or more, five or more, six or more, seven or more, eight or more, nine or more, ten or more, eleven or more, or twelve yield, height, and/or lodging QTLs identified herein to generate a population of pea plants or seeds with a desirable yield, height, and/or lodging phenotype.

In one embodiment, this disclosure provides a method for introgressing an allele of a polymorphic locus conferring a yield, height, and/or lodging phenotype. In specific embodiments, the polymorphic locus corresponds to, or comprises any marker within, the genomic region between positions: 386683682-424059129 of chromosome 2; 57407964-130197960 of chromosome 5, 541653217-end of chromosome 5, 387250594-427410060 of chromosome 2; 547677746-end of chromosome 5; start-17888751 of chromosome 6; 354324649-402078389 of chromosome 2; 18873917-69320372 of chromosome 3; 273327332-422042733 of chromosome 3; 118944413-200876233 of chromosome 4; 17641704-56905305 of chromosome 6; 79787890-153689044 of chromosome 7; 91738904-165762735 of chromosome 7; 207998578-360550624 of chromosome 7; scaffold00011; 73778015-347862320 of chromosome 1 LG6; 287196406-405820442 of chromosome 2 LG1; scaffold05541; 22316149-374998060 of chromosome 4 LG4; 35018291-496475925 of chromosome 5 LG3; 4102 if scaffold00839; 332130985-426328693 of chromosome 6 LG2; 8316115-481251260 of chromosome 7 LG7; scaffold02229; 122338217-340372022 of chromosome 1 LG6; 12117951-41766109 of chromosome 2 LG1; 55052537-420398638 of chromosome 3 LG5; scaffold06018; scaffold03592; super-scaffold8606; 47145729-440853776 of chromosome 4 LG4; scaffold02661; 3579419-570360377 of chromosome 5LG3; super-scaffold9678; 2560210-348314640 of chromosome 6 LG2; 191955823-63397608 of chromosome 7 LG7; 63453702 of chromosome 1 LG6; 96628783-415226641 of chromosome 2 LG1; scaffold05147; 403366757 of chromosome 4 LG4; scaffold00254; 811842-294741093 of chromosome 3 LG5; scaffold03249; 173468835-440508849 of chromosome 4 LG4; 362378-229071963 of chromosome 5 LG3; scaffold01526; 157000374-469077464 of chromosome 6 LG2; scaffold01649; or 750954-487220871 of chromosome 7 LG7 of a *Pisum sativum* genome. The genomic region described herein can correspond to a respective genomic region, i.e., a genomic region between positions: 386683682-424059129 of chromosome 2; 57407964-130197960 of chromosome 5; 541653217-end of chromosome 5; 387250594-427410060 of chromosome 2; 547677746-end of chromosome 5; start-17888751 of chromosome 6; 354324649-402078389 of chromosome 2; 18873917-69320372 of chromosome 3; 273327332-422042733 of chromosome 3; 118944413-200876233 of chromosome 4; 17641704-56905305 of chromosome 6; 79787890-153689044 of chromosome 7; 91738904-165762735 of chromosome 7; 207998578-360550624 of chromosome 7; scaffold00011; 73778015-347862320 of chromosome 1 LG6; 287196406-405820442 of chromosome 2 LG1; scaffold05541; 22316149-374998060 of chromosome 4 LG4; 35018291-496475925 of chromosome 5 LG3; 4102 if scaffold00839; 332130985-426328693 of chromosome 6 LG2; 8316115-481251260 of chromosome 7 LG7; scaffold02229; 122338217-340372022 of chromosome 1 LG6; 12117951-41766109 of chromosome 2 LG1; 55052537-420398638 of chromosome 3 LG5; scaffold06018; scaffold03592; super-scaffold8606; 47145729-440853776 of chromosome 4 LG4; scaffold02661; 3579419-570360377 of chromosome 5LG3; super-scaffold9678; 2560210-348314640 of chromosome 6 LG2; 191955823-63397608 of chromosome 7 LG7; 63453702 of chromosome 1 LG6; 96628783-415226641 of chromosome 2 LG1; scaffold05147; 403366757 of chromosome 4 LG4; scaffold00254; 811842-294741093 of chromosome 3 LG5; scaffold03249; 173468835-440508849 of chromosome 4 LG4; 362378-229071963 of chromosome 5 LG3; scaffold01526; 157000374-469077464 of chromosome 6 LG2; scaffold01649; or 750954-487220871 of chromosome 7 LG7 of the *Pisum sativum* Cameor vla reference genome. The marker within the polymorphic locus can be an SNP marker or a deletion marker.

In specific embodiments, the yield, height, and/or lodging QTL of the present invention may be introduced into an elite *Pisum sativum* variety. An "elite" variety as used herein refers to a variety of the plant that has one or more desirable traits, such as high yield, low lodging, high content of protein, oil, or other nutrients, improved flavor, or increased tolerance to disease or environmental pressures.

A population of pea plants or seeds is provided that is produced by any method disclosed herein. In specific embodiments, the population of pea plants or seeds comprises a mean yield and/or height that is greater than that of a control population, and/or a mean lodging that is lower than that of a control population. For example, a pea plant or pea seed produced by the methods provided herein can have greater yield than a reference or commodity pea seed or reference or commodity pea plant. The population of pea plants or seeds provided herein can have yield that is at least 10, 11, 12, 13, 14, 15, 16, 17, 18, 19, 20, or more bushels/acre greater than that of a reference pea plant or seed or a reference population of pea plants or seeds. Methods provided herein can produce a high yield pea plant or seed, or a high yield pea plant or seed population that has an average yield of 40, 41, 42, 43, 44, 45, 46, 47, 48, 49, 50, 51, 52, 53, 54, 55, or more bushels/acre. In particular embodiment the pea plant or seed population provided herein comprises an average yield of at least 49, 50, or 51 bushels/acre. In contrast, commodity peas may have yield of 25, 26, 27, 28, 29, 30, 31, 32, 33, 34, or 35 bushels/acre.

A pea plant produced by the methods provided herein can be taller than a reference or commodity pea plant. The population of pea plants or seeds provided herein can be taller by at least 5, 6, 7, 8, 9, 10, 11, 12, 13, 14, 15, or more inches than that of a reference pea plant or a reference population of pea plants. Methods provided herein can produce a high height pea plant, or a high height pea plant population that has an average height of 35, 36, 37, 38, 39, 40, 41, 42, or more inches. In particular embodiment the pea population provided herein comprises an average height of at least 35-40 inches. In contrast, commodity peas may have height of 20, 21, 22, 23, 24, 25, 26, or 27 inches.

A pea plant produced by the methods provided herein can have less lodging than a reference or commodity pea plant. The population of pea plants or seeds provided herein can have less lodging by at least 1.5, 1.6, 1.7, 1.8, 1.9, 2.0, 2.1, 2.2, 2.3, 2.4, 2.5, or more lodging unit than that of a reference pea plant or seed or a reference population of pea plants or seeds. Methods provided herein can produce a low lodging pea plant, or a low lodging pea plant population that has an average lodging unit of 2.8, 2.9, 3.0, 3.1, 3.2, 3.3, or 3.4. In particular embodiment the pea population provided herein comprises an average lodging unit of 3.1 or 3.2. In contrast, commodity peas may have a lodging unit of 4.9, 5.0, 5.1, 5.2, 5.3, 5.4, or 5.5.

The reference pea plant or seed can be a commodity pea plant or commodity pea seed, a pea plant or seed that has not been bred using the methods provided herein, or a pea plant or seed that does not contain the yield, height, and/or lodging QTLs provided herein. In specific embodiments, the population of pea plants or seeds provided herein has average yield that is greater by at least 13 bushels per acre, average height that is greater by at least 8 inches, and/or average lodging that is lower by at least 1.5 lodging units relative to a control population of pea plants or seeds not produced by the methods provided herein, e.g., a control population of pea plants or seeds without one or more yield, height, and/or lodging alleles provided herein.

In some embodiments, the population of pea plants or seeds provided herein has average protein or average oil content that is at least 90%, 91%, 92%, 93%, 94%, 95%, 96%, 97%, 98%, 99%, 100%, or more relative to a control population of pea plants or seeds without said one or more yield, height, and/or lodging alleles.

In some embodiments, the population of pea plants or seeds comprises at least one yield, height, and/or lodging QTL provided herein, e.g., one or more of yield chr2-2 (Ps02_417306163), yield chr5-1 (Ps05_117545329), yield chr5-2 (Ps05_604790207), lodging chr2-1 (Ps02_ 415727951), lodging chr5-1 (Ps05_ 608256598), lodging chr6-1 (Ps06_ 5054240), height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364 at a greater frequency than the occurrence of the same yield, height, and/or lodging QTL in a population of pea plants or seeds not produced by the methods disclosed herein.

In specific embodiments, a population of pea seeds, pea seed product, or pea protein product (e.g., pea protein concentrate, pea protein isolate, or pea protein) is provided herein comprising at least one yield, height, and/or lodging QTL disclosed herein at a greater frequency than a control pea seed population or pea protein composition. In some embodiments, a control pea plant or pea seed population or pea protein composition is a population produced by methods without assaying for or introgressing a yield, height, and/or lodging molecular marker, such as those yield, height, and/or lodging molecular markers disclosed herein. The pea seeds, plants, and protein or seed compositions disclosed herein, e.g., produced from the pea plants or seeds provided herein having a desirable yield, height, and/or lodging phenotype, need not contain and need not be produced from a population of plants that exclusively contains a yield, height, and/or lodging molecular marker disclosed herein.

### 2.3 Detection/identification of yield, height, and/or lodging markers and QTLs

The detection of polymorphic sites in a sample of DNA, RNA, or cDNA may be facilitated through the use of nucleic acid amplification methods. Such methods specifically increase the concentration of polynucleotides that span the polymorphic site, or include that site and sequences located either distal or proximal to it. Such amplified molecules can be readily detected by gel electrophoresis or other means.

In certain embodiments of the method described herein, genotyping comprises assaying a single nucleotide polymorphism (SNP) marker. SNPs can be assayed and characterized using any of a variety of methods. Such methods include the direct or indirect sequencing of the site, the use of restriction enzymes where the respective alleles of the site create or destroy a restriction site, the use of allele-specific hybridization probes, the use of antibodies that are specific for the proteins encoded by the different alleles of the polymorphism, or by other biochemical interpretation. SNPs can be sequenced using a variation of the chain termination method (Sanger et al., Proc. Natl. Acad. Sci. (U.S.A.) 74: 5463-5467 (1977)) in which the use of radioisotopes are replaced with fluorescently-labeled dideoxy nucleotides and subjected to capillary based automated sequencing (U.S. Pat. No. 5,332,666, the entirety of which is herein incorporated by reference; U.S. Pat. No. 5,821,058, the entirety of which is herein incorporated by reference). Automated sequencers are available from, for example, Applied Biosystems, Foster City, Calif. (3730x1 DNA Analyzer), Beckman Coulter, Fullerton, Calif. (CEQ^{™} 8000 Genetic Analysis System) and LI-COR, Inc., Lincoln, Nebr. (4300 DNA Analysis System).

Approaches for analyzing SNPs can be categorized into two groups. The first group is based on primer-extension assays, such as solid-phase minisequencing or pyrosequencing. In the solid-phase minisequencing method, a DNA polymerase is used specifically to extend a primer that anneals immediately adjacent to the variant nucleotide. A single labeled nucleoside triphosphate complementary to the nucleotide at the variant site is used in the extension reaction. Only those sequences that contain the nucleotide at the variant site will be extended by the polymerase. A primer array can be fixed to a solid support wherein each primer is contained in four small wells, each well being used for one of the four nucleoside triphosphates present in DNA. Template DNA or RNA from each test organism is put into each well and allowed to anneal to the primer. The primer is then extended one nucleotide using a polymerase and a labeled di-deoxy nucleotide triphosphate. The completed reaction can be imaged using devices that are capable of detecting the label which can be radioactive or fluorescent. Using this method several different SNPs can be visualized and detected (Syvänen et al., Hum. Mutat. 13: 1-10 (1999)). The pyrosequencing technique is based on an indirect bioluminometric assay of the pyrophosphate (PPi) that is released from each dNTP upon DNA chain elongation. Following Klenow polymerase mediated base incorporation, Ppi is released and used as a substrate, together with adenosine 5-phosphosulfate (APS), for ATP sulfurylase, which results in the formation of ATP. Subsequently, the ATP accomplishes the conversion of luciferin to its oxi-derivative by the action of luciferase. The ensuing light output becomes proportional to the number of added bases, up to about four bases. To allow processivity of the method dNTP excess is degraded by apyrase, which is also present in the starting reaction mixture, so that only dNTPs are added to the template during the sequencing procedure (Alderborn et al., Genome Res. 10: 1249-1258 (2000)). An example of an instrument designed to detect and interpret the pyrosequencing reaction is available from Biotage, Charlottesville, Va. (PyroMark MD).

Another SNP detection method based on primer-extension assays is commonly referred to as the GOOD assay. The GOOD assay (Sauer et al., Nucleic Acids Res. 28: e100 (2000)) is an allele-specific primer extension protocol that employs MALDI-TOF (matrix-assisted laser desorption/ionization time-of-flight) mass spectrometry. The region of DNA containing an SNP is amplified first by PCR amplification. Residual dNTPs are destroyed using an alkaline phosphatase. Allele-specific products are then generated using a specific primer, a conditioned set of a-S-dNTPs and α-S-ddNTPs and a fresh DNA polymerase in a primer extension reaction. Unmodified DNA is removed by 5'phosphodiesterase digestion and the modified products are alkylated to increase the detection sensitivity in the mass spectrometric analysis. All steps are carried out in a single vial at the lowest practical sample volume and require no purification. The extended reaction can be given a positive or negative charge and is detected using mass spectrometry (Sauer et al., Nucleic Acids Res. 28: e13 (2000)). An instrument in which the GOOD assay is analyzed is for example, the AUTOFLEX^{®} MALDI-TOF system from Bruker Daltonics (Billerica, Mass.).

In some embodiments of the method described herein, genotyping comprises assaying a deletion marker. Any method known in the art can be used to identify a region of the genome that is missing a given position, including but not limited to PCR, RFLP, probe-based detection methods, and sequencing methods, among others.

In one embodiment of the method described herein, genotyping comprises the use of an oligonucleotide probe. The use of an oligonucleotide probe is based on recognition of heteroduplex DNA molecules and includes oligonucleotide hybridization, TAQ-MAN^{®} assays, molecular beacons, electronic dot blot assays and denaturing high-performance liquid chromatography. Oligonucleotide hybridizations can be performed in mass using micro-arrays (Southern, Trends Genet. 12: 110-115 (1996)). TAQ-MAN^{®} assays, or Real Time PCR, detects the accumulation of a specific PCR product by hybridization and cleavage of a double-labeled fluorogenic probe during the amplification reaction. A TAQ-MAN^{®} assay includes four oligonucleotides, two of which serve as PCR primers and generate a PCR product encompassing the polymorphism to be detected. The other two are allele-specific fluorescence-resonance-energy-transfer (FRET) probes. FRET probes incorporate a fluorophore and a quencher molecule in close proximity so that the fluorescence of the fluorophore is quenched. The signal from a FRET probes is generated by degradation of the FRET oligonucleotide, so that the fluorophore is released from proximity to the quencher, and is thus able to emit light when excited at an appropriate wavelength. In the assay, two FRET probes bearing different fluorescent reporter dyes are used, where a unique dye is incorporated into an oligonucleotide that can anneal with high specificity to only one of the two alleles. Useful reporter dyes include 6-carboxy-4,7,2',7'-tetrachlorofluorecein (TET), 2'-chloro-7'-phenyl-1,4-dichloro-6-carboxyfluorescein (VIC) and 6-carboxyfluorescein phosphoramidite (FAM). A useful quencher is 6-carboxy-N,N,N',N'-tetramethylrhodamine (TAMRA). Annealed (but not non-annealed) FRET probes are degraded by TAQ DNA polymerase as the enzyme encounters the 5' end of the annealed probe, thus releasing the fluorophore from proximity to its quencher. Following the PCR reaction, the fluorescence of each of the two fluorescers, as well as that of the passive reference, is determined fluorometrically. The normalized intensity of fluorescence for each of the two dyes will be proportional to the amounts of each allele initially present in the sample, and thus the genotype of the sample can be inferred. An example of an instrument used to detect the fluorescence signal in TAQ-MAN^{®} assays, or Real Time PCR are the 7500 Real-Time PCR System (Applied Biosystems, Foster City, Calif.).

Molecular beacons are oligonucleotide probes that form a stem-and-loop structure and possess an internally quenched fluorophore. When they bind to complementary targets, they undergo a conformational transition that turns on their fluorescence. These probes recognize their targets with higher specificity than linear probes and can easily discriminate targets that differ from one another by a single nucleotide. The loop portion of the molecule serves as a probe sequence that is complementary to a target nucleic acid. The stem is formed by the annealing of the two complementary arm sequences that are on either side of the probe sequence. A fluorescent moiety is attached to the end of one arm and a nonfluorescent quenching moiety is attached to the end of the other arm. The stem hybrid keeps the fluorophore and the quencher so close to each other that the fluorescence does not occur. When the molecular beacon encounters a target sequence, it forms a probe-target hybrid that is stronger and more stable than the stem hybrid. The probe undergoes spontaneous conformational reorganization that forces the arm sequences apart, separating the fluorophore from the quencher, and permitting the fluorophore to fluoresce (Bonnet et al., 1999). The power of molecular beacons lies in their ability to hybridize only to target sequences that are perfectly complementary to the probe sequence, hence permitting detection of single base differences (Kota et al., Plant Mol. Biol. Rep. 17: 363-370 (1999)). Molecular beacon detection can be performed for example, on the Mx4000^{®} Multiplex Quantitative PCR System from Stratagene (La Jolla, Calif.).

In one embodiment, the SNP marker described in the methods provided herein is capable of being identified by a corresponding nucleic acid molecule that comprises at least 15 nucleotides that include or are immediately adjacent to the SNP. The nucleic acid molecule described above is at least at least 90% (90%, 91%, 92%, 93%, 94%, 95%, 96%, 97%, 98%, 99%, or 100%) identical to a sequence of the same number of consecutive nucleotides in either strand of DNA that include or are immediately adjacent to the SNP. Likewise, the deletion marker disclosed herein is capable of being identified by a corresponding nucleic acid molecule that comprises at least 15 nucleotides that include or are immediately adjacent to the deletion, or by a nucleic acid molecule that only binds to the unique junction formed by the deletion event.

In one embodiment, the disclosure provides an isolated nucleic acid molecule for detecting a yield, height, and/or lodging molecular marker in pea DNA. The nucleic acid molecule comprises at least 15 nucleotides that include or are immediately adjacent to the marker, wherein the nucleic acid molecule is at least 90% (91%, 92%, 93%, 94%, 95%, 96%, 97%, 98%, 99%, or 100%) identical to a sequence of the same number of consecutive nucleotides in either strand of DNA that include or are immediately adjacent to the marker.

In some embodiments, the yield, height, and/or lodging molecular marker is an SNP marker, and the SNP marker is selected from the group consisting of T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A or G at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; G at position 195183596 of chromosome 7LG7; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of the *Pisum sativum* genome (e.g., positions on chromosomes corresponding to the respective positions of the *Pisum sativum* Cameor v1a reference genome).

The nucleic acid molecule provided herein can further include a detectable label. In some embodiments, the detectable label is a fluorescent label or a radioactive label.

The electronic dot blot assay uses a semiconductor microchip comprised of an array of microelectrodes covered by an agarose permeation layer containing streptavidin. Biotinylated amplicons are applied to the chip and electrophoresed to selected pads by positive bias direct current, where they remain embedded through interaction with streptavidin in the permeation layer. The DNA at each pad is then hybridized to mixtures of fluorescently labeled allele-specific oligonucleotides. Single base pair mismatched probes can then be preferentially denatured by reversing the charge polarity at individual pads with increasing amperage. The array is imaged using a digital camera and the fluorescence quantified as the amperage is ramped to completion. The fluorescence intensity is then determined by averaging the pixel count values over a region of interest (Gilles et al., Nature Biotech. 17: 365-370 (1999)).

A more recent application based on recognition of heteroduplex DNA molecules uses denaturing high-performance liquid chromatography (DHPLC). This technique represents a highly sensitive and fully automated assay that incorporates a Peltier-cooled 96-well autosampler for high-throughput SNP analysis. It is based on an ion-pair reversed-phase high performance liquid chromatography method. The heart of the assay is a polystyrene-divinylbenzene copolymer, which functions as a stationary phase. The mobile phase is composed of an ion-pairing agent, triethylammonium acetate (TEAA) buffer, which mediates the binding of DNA to the stationary phase, and an organic agent, acetonitrile (ACN), to achieve subsequent separation of the DNA from the column. A linear gradient of CAN allows the separation of fragments based on the presence of heteroduplexes. DHPLC thus identifies mutations and polymorphisms that cause heteroduplex formation between mismatched nucleotides in double-stranded PCR-amplified DNA. In a typical assay, sequence variation creates a mixed population of heteroduplexes and homoduplexes during reannealing of wild-type and mutant DNA. When this mixed population is analyzed by DHPLC under partially denaturing temperatures, the heteroduplex molecules elute from the column prior to the homoduplex molecules, because of their reduced melting temperatures (Kota et al., Genome 44: 523-528 (2001)). An example of an instrument used to analyze SNPs by DHPLC is the WAVE^{®} HS System from Transgenomic, Inc. (Omaha, Nebr.).

A microarray-based method for high-throughput monitoring of plant gene expression can be utilized as a genetic marker system. This 'chip'-based approach involves using microarrays of nucleic acid molecules as gene-specific hybridization targets to quantitatively or qualitatively measure expression of plant genes (Schena et al., Science 270:467-470 (1995), the entirety of which is herein incorporated by reference; Shalon, Ph.D. Thesis. Stanford University (1996), the entirety of which is herein incorporated by reference). Every nucleotide in a large sequence can be queried at the same time. Hybridization can be used to efficiently analyze nucleotide sequences. Such microarrays can be probed with any combination of nucleic acid molecules. Particularly preferred combinations of nucleic acid molecules to be used as probes include a population of mRNA molecules from a known tissue type or a known developmental stage or a plant subject to a known stress (environmental or man-made) or any combination thereof (e.g. mRNA made from water stressed leaves at the 2 leaf stage). Expression profiles generated by this method can be utilized as markers.

Polymorphisms can also be identified by Single Strand Conformation Polymorphism (SSCP) analysis. SSCP is a method capable of identifying most sequence variations in a single strand of DNA, typically between 150 and 250 nucleotides in length (Elles, Methods in Molecular Medicine: Molecular Diagnosis of Genetic Diseases, Humana Press (1996); Orita et al., Genomics 5: 874-879 (1989)). Under denaturing conditions, a single strand of DNA will adopt a conformation that is uniquely dependent on its sequence conformation. This conformation usually will be different, even if only a single base is changed. Most conformations have been reported to alter the physical configuration or size sufficiently to be detectable by electrophoresis.

In one embodiment of the method described herein, the oligonucleotide probe is adjacent to a polymorphic nucleotide position in the yield, height, and/or lodging QTL. For the purpose of QTL mapping, the markers included must be diagnostic of origin in order for inferences to be made about subsequent populations. SNP markers are ideal for mapping because the likelihood that a particular SNP allele is derived from independent origins in the extant populations of a particular species is very low. As such, SNP markers are useful for tracking and assisting introgression of QTLs, particularly in the case of haplotypes. In one embodiment of the method described herein, genotyping comprises detecting a haplotype.

GEMMA GWAS methods can be used to identify the top genomic regions (QTL) associated with a desirable yield, height, and/or lodging trait.

In some embodiments, the method further includes determining the yield, height, and/or lodging of the second population of pea plants or seeds, wherein the second population of pea plants or seeds having the one or more yield, height, and/or lodging alleles have greater yield, greater height, and/or less lodging when compared to a control population of pea plants or seeds lacking the one or more yield, height, and/or lodging alleles. In some embodiments, the second population of pea plants has a greater frequency of the at least one yield, height, and/or lodging molecular marker provided herein than said first population of pea plants. Determining yield, height, or lodging in a seed or plant is well known to the person of skill in the art and any such methods known to a skilled artisan may be used.

The genetic linkage of additional marker molecules can be established by a gene mapping model such as, without limitation, the flanking marker model reported by Lander and Botstein, Genetics, 121:185-199 (1989), and the interval mapping, based on maximum likelihood methods described by Lander and Botstein, Genetics, 121:185-199 (1989), and implemented in the software package MAPMAKER/QTL (Lincoln and Lander, Mapping Genes Controlling Quantitative Traits Using MAPMAKER/QTL, Whitehead Institute for Biomedical Research, Massachusetts, (1990). Additional software includes Qgene, Version 2.23 (1996), Department of Plant Breeding and Biometry, 266 Emerson Hall, Cornell University, Ithaca, N.Y., the manual of which is herein incorporated by reference in its entirety). Use of Qgene software is a particularly preferred approach.

A maximum likelihood estimate (MLE) for the presence of a marker is calculated, together with an MLE assuming no QTL effect, to avoid false positives. A log10 of an odds ratio (LOD) is then calculated as: LOD=log10 (MLE for the presence of a QTL/MLE given no linked QTL). The LOD score essentially indicates how much more likely the data are to have arisen assuming the presence of a QTL versus in its absence. The LOD threshold value for avoiding a false positive with a given confidence, say 95%, depends on the number of markers and the length of the genome. Graphs indicating LOD thresholds are set forth in Lander and Botstein, Genetics, 121:185-199 (1989), and further described by Arús and Moreno-Gonzalez, Plant Breeding, Hayward, Bosemark, Romagosa (eds.) Chapman & Hall, London, pp. 314-331 (1993).

Additional models can be used. Many modifications and alternative approaches to interval mapping have been reported, including the use of non-parametric methods (Kruglyak and Lander, Genetics, 139:1421-1428 (1995), the entirety of which is herein incorporated by reference). Multiple regression methods or models can also be used, in which the trait is regressed on a large number of markers (Jansen, Biometrics in Plant Breed, van Oijen, Jansen (eds.) Proceedings of the Ninth Meeting of the Eucarpia Section Biometrics in Plant Breeding, The Netherlands, pp. 116-124 (1994); Weber and Wricke, Advances in Plant Breeding, Blackwell, Berlin, 16 (1994)). Procedures combining interval mapping with regression analysis, whereby the phenotype is regressed onto a single putative QTL at a given marker interval, and at the same time onto a number of markers that serve as 'cofactors,' have been reported by Jansen and Stam, Genetics, 136:1447-1455 (1994) and Zeng, Genetics, 136:1457-1468 (1994). Generally, the use of cofactors reduces the bias and sampling error of the estimated QTL positions (Utz and Melchinger, Biometrics in Plant Breeding, van Oijen, Jansen (eds.) Proceedings of the Ninth Meeting of the Eucarpia Section Biometrics in Plant Breeding, The Netherlands, pp. 195-204 (1994), thereby improving the precision and efficiency of QTL mapping (Zeng, Genetics, 136:1457-1468 (1994)). These models can be extended to multi-environment experiments to analyze genotype-environment interactions (Jansen et al., Theo. Appl. Genet. 91:33-37 (1995).

Selection of appropriate mapping populations is important to map construction. The choice of an appropriate mapping population depends on the type of marker systems employed (Tanksley et al., Molecular mapping of plant chromosomes. chromosome structure and function: Impact of new concepts J. P. Gustafson and R. Appels (eds.). Plenum Press, New York, pp. 157-173 (1988), the entirety of which is herein incorporated by reference). Consideration must be given to the source of parents (adapted vs. exotic) used in the mapping population. Chromosome pairing and recombination rates can be severely disturbed (suppressed) in wide crosses (adapted×exotic) and generally yield greatly reduced linkage distances. Wide crosses will usually provide segregating populations with a relatively large array of polymorphisms when compared to progeny in a narrow cross (adapted×adapted).

An F2 population is the first generation of selfing after the hybrid seed is produced. Usually a single F1 plant is selfed to generate a population segregating for all the genes in Mendelian (1:2:1) fashion. Maximum genetic information is obtained from a completely classified F2 population using a codominant marker system (Mather, Measurement of Linkage in Heredity: Methuen and Co., (1938), the entirety of which is herein incorporated by reference). In the case of dominant markers, progeny tests (e.g., F3, BCF2) are required to identify the heterozygotes, thus making it equivalent to a completely classified F2 population. However, this procedure is often prohibitive because of the cost and time involved in progeny testing. Progeny testing of F2 individuals is often used in map construction where phenotypes do not consistently reflect genotype (e.g. disease resistance) or where trait expression is controlled by a QTL. Segregation data from progeny test populations (e.g. F3 or BCF2) can be used in map construction. Marker-assisted selection can then be applied to cross progeny based on marker-trait map associations (F2, F3), where linkage groups have not been completely disassociated by recombination events (i.e., maximum disequilibrium).

In certain embodiments of the method described herein, genotyping comprises assaying for a deletion marker. As with SNP markers, deletion markers can be identified or detected using standard nucleotide amplification techniques and/or oligonucleotide probes. In specific embodiments, deletion makers can be detected by amplifying a region comprising the complete deletion using primers located upstream (5') and downstream (3') of the anticipated deletion. Oligonucleotide probes can be designed to specifically detect a deletion marker by detecting the junction of the ligation of the upstream (5') and downstream (3') regions of the anticipated deletion. Oligo nucleotide probes disclosed herein can be labelled with any detection label used in the art including, but not limited to, fluorescent probes and radiolabeled probes.

### 2.4. Breeding of yield, height, and/or lodging pea plants

Yield, height, and/or lodging pea plants of the present disclosure can be part of or generated from a breeding program. The choice of breeding method depends on the mode of plant reproduction, the heritability of the trait(s) being improved, and the type of cultivar used commercially (e.g., F1 hybrid cultivar, pureline cultivar, etc.). A cultivar is a race or variety of a plant that has been created or selected intentionally and maintained through cultivation.

Descriptions of breeding methods that are commonly used for different crops can be found in one of several reference books, see, e.g., Allard, Principles of Plant Breeding, John Wiley & Sons, NY, U. of CA, Davis, Calif., 50-98 (1960); Simmonds, Principles of Crop Improvement, Longman, Inc., NY, 369-399 (1979); Sneep and Hendriksen, Plant breeding Perspectives, Wageningen (ed), Center for Agricultural Publishing and Documentation (1979); Fehr, Peas: Improvement, Production and Uses, 2nd Edition, Monograph, 16:249 (1987); Fehr, Principles of Variety Development, Theory and Technique, (Vol. 1) and Crop Species Pea (Vol. 2), Iowa State Univ., Macmillan Pub. Co., NY, 360-376 (1987).

Selected, non-limiting approaches for breeding the plants of the present invention are set forth below. A breeding program can be enhanced using marker assisted selection (MAS) of the progeny of any cross. It is further understood that any commercial and non-commercial cultivars can be utilized in a breeding program. Factors such as, for example, emergence vigor, vegetative vigor, stress tolerance, disease resistance, branching, flowering, seed set, seed size, seed density, standability, and threshability etc. will generally dictate the choice.

For highly heritable traits, a choice of superior individual plants evaluated at a single location will be effective, whereas for traits with low heritability, selection should be based on mean values obtained from replicated evaluations of families of related plants. Popular selection methods commonly include pedigree selection, modified pedigree selection, mass selection, and recurrent selection. In a preferred embodiment a backcross or recurrent breeding program is undertaken.

The complexity of inheritance influences choice of the breeding method. Backcross breeding can be used to transfer one or a few favorable genes for a highly heritable trait into a desirable cultivar. This approach has been used extensively for breeding disease-resistant cultivars. Various recurrent selection techniques are used to improve quantitatively inherited traits controlled by numerous genes. The use of recurrent selection in self-pollinating crops depends on the ease of pollination, the frequency of successful hybrids from each pollination event, and the number of hybrid offspring from each successful cross.

Breeding lines can be tested and compared to appropriate standards in environments representative of the commercial target area(s) for two or more generations. The best lines are candidates for new commercial cultivars; those still deficient in traits may be used as parents to produce new populations for further selection.

One method of identifying a superior plant is to observe its performance relative to other experimental plants and to a widely grown standard cultivar. If a single observation is inconclusive, replicated observations can provide a better estimate of its genetic worth. A breeder can select and cross two or more parental lines, followed by repeated selfing and selection, producing many new genetic combinations.

The development of new pea cultivars requires the development and selection of pea varieties, the crossing of these varieties and selection of superior hybrid crosses. The hybrid seed can be produced by manual crosses between selected male-fertile parents or by using male sterility systems. Hybrids are selected for certain single gene traits such as pod color, flower color, seed yield, pubescence color or herbicide resistance which indicate that the seed is truly a hybrid. Additional data on parental lines, as well as the phenotype of the hybrid, influence the breeder's decision whether to continue with the specific hybrid cross.

Pedigree breeding and recurrent selection breeding methods can be used to develop cultivars from breeding populations. Breeding programs combine desirable traits from two or more cultivars or various broad-based sources into breeding pools from which cultivars are developed by selfing and selection of desired phenotypes. New cultivars can be evaluated to determine which have commercial potential.

Pedigree breeding is used commonly for the improvement of self-pollinating crops. Two parents who possess favorable, complementary traits (e.g., high yield, and/or low lodging trait with optional high height or low height trait) are crossed to produce an F1. An F2 population is produced by selfing one or several F1's. Selection of the best individuals in the best families is selected. Replicated testing of families can begin in the F4 generation to improve the effectiveness of selection for traits with low heritability. At an advanced stage of inbreeding (i.e., F6 and F7), the best lines or mixtures of phenotypically similar lines are tested for potential release as new cultivars.

Backcross breeding has been used to transfer genes for a simply inherited, highly heritable trait into a desirable homozygous cultivar or inbred line, which is the recurrent parent. The source of the trait to be transferred is called the donor parent. The resulting plant is expected to have the attributes of the recurrent parent (e.g., cultivar) and the desirable trait transferred from the donor parent. After the initial cross, individuals possessing the phenotype of the donor parent are selected and repeatedly crossed (backcrossed) to the recurrent parent. The resulting parent is expected to have the attributes of the recurrent parent (e.g., cultivar) and the desirable trait transferred from the donor parent.

The single-seed descent procedure in the strict sense refers to planting a segregating population, harvesting a sample of one seed per plant, and using the one-seed sample to plant the next generation. When the population has been advanced from the F2 to the desired level of inbreeding, the plants from which lines are derived will each trace to different F2 individuals. The number of plants in a population declines each generation due to failure of some seeds to germinate or some plants to produce at least one seed. As a result, not all of the F2 plants originally sampled in the population will be represented by a progeny when generation advance is completed.

In a multiple-seed procedure, pea breeders commonly harvest one or more pods from each plant in a population and thresh them together to form a bulk. Part of the bulk is used to plant the next generation and part is put in reserve. The procedure has been referred to as modified single-seed descent or the pod-bulk technique.

The multiple-seed procedure has been used to save labor at harvest. It is considerably faster to thresh pods with a machine than to remove one seed from each by hand for the single-seed procedure. The multiple-seed procedure also makes it possible to plant the same number of seed of a population each generation of inbreeding.

Descriptions of other breeding methods that are commonly used for different traits and crops can be found in one of several reference books (e.g., Fehr, Principles of Cultivar Development Vol. 1, pp. 2-3 (1987)).

### 2.5 Plants, plant parts, plant cells, and plant products

Disclosed herein are yield, height, and/or lodging pea plants, plant parts (e.g., juice, pulp, seed, grain, fruit, flowers, nectar, embryos, pollen, ovules, leaves, stems, branches, bark, kernels, ears, cobs, husks, stalks, roots, root tips, anthers, etc.), or plant products produced by the methods provided herein. Progeny, variants, and mutants of the produced plants are also included within the scope of the invention, provided that they comprise the yield, height, and/or lodging phenotype.

"Plant products," as used herein, refers to any product or composition produced from the plant, including any oil products, sugar products, fiber products, protein products (such as protein concentrate, protein isolate, flake, or other protein product), seed products, seed hulls, meal, or flour, for a food, feed, aqua, or industrial product, plant extract (e.g., sweetener, antioxidants, alkaloids, etc.), plant concentrate (e.g., whole plant concentrate or plant part concentrate), plant powder (e.g., formulated powder, such as formulated plant part powder (e.g., seed flour)), plant biomass (e.g., dried biomass, such as crushed and/or powdered biomass), grains, plant protein composition, plant oil composition, and food and beverage products containing plant compositions (e.g., plant parts, plant extract, plant concentrate, plant powder, plant protein, plant oil, and plant biomass) described herein. Plant parts and plant products provided herein can be intended for human or animal consumption.

As used herein, a "protein product" or "protein composition" refers to any protein composition or product isolated, extracted, and/or produced from plants or plant parts (e.g., seed) and includes isolates, concentrates, and flours, e.g., pea protein composition, pea protein concentrate (SPC), pea protein isolate (SPI), pea flour, flake, white flake, texturized vegetable protein (TVP), or textured pea protein (TPP)). A protein composition can be a concentrated protein solution (e.g., yellow pea protein concentrate solution) in which the protein is in a higher concentration than the protein in the plant from which the protein composition is derived.

"White flake protein" as used herein refers to a protein composition obtained by de-hulling, flaking, and defattening plants or plant parts (e.g., legume plants or plant parts) by solvent (e.g., hexane) extraction, with limited use of heat to run off the solvent (Lusas and Riaz, 1995). White flake protein is an intermediate product in the production of plant protein concentrates and isolates. In contrast to conventional toasted plant meal (e.g., pea protein meal), white flakes contains undenaturated proteins due to the very mild heat treatment. Thus, little or no reduction of protease inhibitors would be expected. The undenaturated proteins in white flakes may be advantageous in supporting binding properties during production of the extruded compound feed. White flakes can be used for human and animal consumption, including as a source of protein in aquaculture feeds for any type of fish or aquatic animal in a farmed or wild environment.

The protein composition can comprise multiple proteins as a result of the extraction or isolation process. In specific embodiments, the protein composition can further comprise stabilizers, excipients, drying agents, desiccating agents, anti-caking agents, or any other ingredient to make the protein fit for the intended purpose. The protein composition can be a solid, liquid, gel, or aerosol and can be formulated as a powder. The protein composition can be extracted in a powder form from a plant and can be processed and produced in different ways, such as: *(i) as an isolate* - through the process of wet fractionation, which has the highest protein concentration; *(ii) as a concentrate* - through the process of dry fractionation, which are lower in protein concentration; and/or *(iii) in textured form* - when it is used in food products as a substitute for other products, such as meat substitution (e.g. a "meat" patty). Protein isolate can be derived from defatted pea flour with a high solubility in water, as measured by the nitrogen solubility index (NSI). The aqueous extraction is carried out at a pH below 9. The extract is clarified to remove the insoluble material and the supernatant liquid is acidified to a pH range of 4-5. The precipitated protein-curd is collected and separated from the whey by centrifuge. The curd can be neutralized with alkali to form the sodium proteinate salt before drying. Protein concentrate can be produced by immobilizing the pea globulin proteins while allowing the soluble carbohydrates, whey proteins, and salts to be leached from the defatted flakes or flour. The protein is retained by one or more of several treatments: leaching with 20-80% aqueous alcohol/solvent, leaching with aqueous acids in the isoelectric zone of minimum protein solubility, pH 4-5; leaching with chilled water (which may involve calcium or magnesium cations), and leaching with hot water of heat-treated defatted protein meal/flour (e.g., pea meal/flour). Any of the process provided herein can result in a product that is 70% protein, 20% carbohydrates (2.7 to 5% crude fiber), 6% ash and about 1% oil, but the solubility may differ. As an example, one ton (t) of defatted pea flakes can yield about 750 kg of pea protein concentrate. "Texturized vegetable protein" (TVP), "Textured vegetable protein," "textured pea protein" (TPP), pea meat, or pea chunks refers to a defatted plant (e.g., pea) flour product, a by-product of extracting plant (e.g., pea) oil. It can be used as a meat analogue or meat extender. It is quick to cook, with a protein content comparable to certain meats. TVP can be produced from any protein-rich seed meal left over from vegetable oil production. A wide range of pulse seeds other than pea, such as lentils, peas, and fava beans, or peanut may be used for TVP production. TVP can be made from pea isolate, flour, or concentrate, and can also be made from cottonseed, wheat, and oats. It is extruded into various shapes (chunks, flakes, nuggets, grains, and strips) and sizes, exiting the nozzle while still hot and expanding as it does so. The defatted thermoplastic proteins are heated to 150-200 °C, which denatures them into a fibrous, insoluble, porous network that can soak up as much as three times its weight in liquids. As the pressurized molten protein mixture exits the extruder, the sudden drop in pressure causes rapid expansion into a puffy solid that is then dried. As much as 50% protein when dry, TVP can be rehydrated at a 2:1 ratio, which drops the percentage of protein to an approximation of ground meat at 16%. TVP can be used as a meat substitute. When cooked together, TVP can help retain more nutrients from the meat by absorbing juices normally lost. Also provided herein are methods of isolating, extracting, or preparing any of the protein compositions or protein products provided herein from plants or plant parts.

Also provided herein are food and/or beverage products containing plant compositions (e.g., plant parts, plant extract, plant concentrate, plant powder, plant protein, and plant biomass) described hereinabove, such as plant compositions derived from the plants or plant parts of the present disclosure. Such food and/or beverage products include, without limitation, shakes, juices, health drinks, alternative meat products (e.g., meatless burger patties, meatless sausages, etc.), alternative egg products (e.g., eggless mayo), and non-dairy products (e.g., non-dairy whipped toppings, non-dairy milk, non-dairy creamer, non-dairy milk shakes, etc. and condiments. A food and/or beverage product that contains plant compositions obtained from plants or plant parts of the present disclosure can have desired traits, compared to a similar or comparable food and/or beverage product that contains plant compositions obtained from a control plant or plant part.

Plant parts (e.g., seeds) and plant products (e.g., plant biomass, seed compositions, protein compositions, food and/or beverage products) produced by the methods provided herein can be meant for consumption by agricultural animals or for use as feed in an agriculture or aquaculture system. In specific embodiments, plant parts and plant products produced according to the methods provided herein include animal feed (e.g., roughages - forage, hay, silage; concentrates - cereal grains, pea cake) intended for consumption by bovine, porcine, poultry, lambs, goats, or any other agricultural animal. In some embodiments, plant parts and plant products produced according to the methods include aquaculture feed for any type of fish or aquatic animal in a farmed or wild environment including, without limitation, trout, carp, catfish, salmon, tilapia, crab, lobster, shrimp, oysters, clams, mussels, and scallops.

Plants, plant parts, or plant products produced by the method of producing a population of pea plants or seeds with a desirable yield, height, and/or lodging phenotype provided herein can have a greater frequency of any of the yield, height, and/or lodging molecular marker provided herein relative to the starting, or control population of pea plants, plant parts, or plant products. Plants, plant parts, or plant products produced by the method of introgressing a yield, height, and/or lodging QTL can have a greater frequency of any of the yield, height, and/or lodging QTLs and/or a desirable yield, height, and/or lodging phenotype relative to the starting, or control population of pea plants, plant parts, or plant products. The yield, height, and/or lodging molecular markers and/or QTLs provided herein include yield chr2-2 (Ps02_417306163), yield chr5-1 (Ps05_117545329), yield chr5-2 (Ps05_604790207), lodging chr2-1 (Ps02_ 415727951), lodging chr5-1 (Ps05_ 608256598), lodging chr6-1 (Ps06_ 5054240), height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03 437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364.

It will be readily apparent to those skilled in the art that other suitable modifications and adaptations of the methods of the invention described herein are obvious and may be made using suitable equivalents without departing from the scope of the invention or the embodiments disclosed herein. Having now described the invention in detail, the same will be more clearly understood by reference to the following examples, which are included for purposes of illustration only and are not intended to be limiting. Unless otherwise noted, all parts and percentages are by dry weight.

### EXAMPLES

### Example 1. Genome-wide association study (GWAS) reveals quantitative trait locus (QTL) for yield, height, and lodging, and protein content

A genotyping-by-sequence (GBS) panel with genome-wide association study (GWAS)-based and stride-selected markers for protein prediction was designed. The GWAS population included 875 breeding lines derived from 14 cultivars and 5 PI lines (NAM population). Lines were sequenced at 1x coverage and aligned against an in-house reference genome, and 47.4 million SNPs were analyzed. Using linkage disequilibrium (LD) pruned GEMMA (Genome-wide Efficient Mixed Model Association), significant peak SNPs (p < 1.00 x 10⁻⁷) associated with yield, height, lodging, and protein content were identified from the GWAS output. Details on the method for GEMMA can be found in Xiang Zhou and Matthew Stephens 2012 Nature Genetics 44, 821-824, herein incorporated by reference in its entirety.

The identified peak SNPs were mapped from the in-house reference genome to Cameor v1a reference genome by BLAST searches. If peak SNP cannot be identified in Cameor, a matching sequence position closest to peak SNP was used. If peak SNP is on a scaffold or non-syntenic chromosome region, the SNP was substituted with the first nucleotide pair of the closest syntenic gene unless otherwise noted. Physical positions of genetic markers that are 10 cM apart from the peak SNP genetic marker were identified. If multiple markers were available with same map position, the one that is furthest away from the peak SNP marker was selected. If no marker was available at exactly 10 cM distance, the next marker more than 10 cM away from the peak SNP marker was selected.

Table 1 summarizes the identified yield, height, and/or lodging QTLs associated with a desirable yield, height, and/or lodging phenotype. Tables 2A and 2B depict yield QTLs in the GWAS population. Tables 3-5 depict phenotypic effect of yield QTLs on seed protein content, yield, lodging, and height. Tables 6A and 6B depict lodging QTLs in the GWAS population. Tables 7-9 depict phenotypic effect of lodging QTLs on seed protein content, yield, lodging, and height. Tables 10A and 10B depict height QTLs in the GWAS population. Tables 11-18 depict phenotypic effect of height QTLs on seed protein content, yield, lodging, and height. Alleles associated with high yield, high height, or low lodging phenotype are shown in bold.

Each marker identified in Tables 2A, 2B, 6A, 6B, 10A, and 10B includes the SNP marker with 100 nucleotide example/representative genomic sequences upstream and downstream of the SNP marker. The sequence upstream and downstream of the SNP may vary. Each marker (such as yield chr2-2, also referred to as Ps02_417306163) provided herein comprises the SNP marker indicated in Tables 2A, 2B, 6A, 6B, 10A, and 10B, with upstream and downstream nucleic acid sequences that have at least 85% sequence identity with the nucleic acid sequence indicated in T Tables 2A, 2B, 6A, 6B, 10A, and 10B for each marker. Each marker listed in Tables 2A, 2B, 6A, 6B, 10A, and 10B (referred to as "marker AA") can also refer to an SNP marker at position 101 of the nucleic acid sequence identified for the marker AA (referred to as "SEQ ID NO: BB") in a genomic region comprising the nucleic acid sequence of SEQ ID NO: BB, or at a corresponding position of a genomic region at least about 50, 100, 150, or 200 nucleotides of which is aligned to SEQ ID NO: BB for maximum homology (e.g., 85%, 86%, 87%, 88%, 89%, 90%, 91%, 92%, 93%, 94%, 95%, 96%, 97%, 98%, or 99% homology) when using standard alignment parameters. For example, the SNP marker identified as yield chr2-2 or Ps02_417306163 can have a sequence that has at least 85% identity with SEQ ID NO: 1. The marker identified as yield chr2-2 or Ps02_417306163 can also refer to an SNP marker at position 101 of a nucleic acid sequence of SEQ ID NO: 1 in a genomic region comprising the nucleic acid sequence of SEQ ID NO: 1, or at a corresponding position of a genomic region at least about 50, 100, 150, or 200 nucleotides of which is aligned to SEQ ID NO: 1 for maximum homology (e.g., 85%, 86%, 87%, 88%, 89%, 90%, 91%, 92%, 93%, 94%, 95%, 96%, 97%, 98%, or 99% homology) when using standard alignment parameters.

In Tables 3-5, 7-9, and 11-18, the phenotypic averages of lines that are homozygous for the reference allele, homozygous for the variant allele, and heterozygous at the SNP marker position were calculated, and the difference between the homozygous alleles were obtained. As shown in Tables 3-5, 7-9, and 11-18, the difference in the phenotypic average between the homozygous alleles corresponded well with the effect size, which is twice the value of beta (effect size) per allele shown in Tables 2A, 2B, 6A, 6B, 10A, and 10B.

**Table 1. Summary of yield, height, and/or lodging QTLs identified in the GWAS population**

| Trait | QTL count | p value range | Effect size range | Pleiotropic effects | Source for desired allele |
|---|---|---|---|---|---|
| Yield | 3 | 2.68 x 10⁻¹⁴ - 3.48 x 10⁻⁸ | 13.64 - 18.34 bushels/acre | lodging, height | cultivars |
| Lodging | 3 | 7.44 x 10⁻²⁴ - 9.76 x 10⁻¹⁶ | 1.67 - 1.97 lodging units | yield, height | cultivars |
| Height | 8 | 4.77 x 10⁻¹⁷ - 3.16 x 10⁻¹⁰ | 8.92 - 13.24 inches | lodging, yield | cultivars |

**Table 2A. Yield QTLs in the GWAS population mapped to the Cameor v1a reference genome**

| QTL | SEQ ID NO: | Peak SNP Cameor | Peak Range Cameor | Size [bp] Cameor | Peak ID Cameor | ID range Cameor | Genetic Map Position Cameor | ~20 cM range (+/-10 cM) Cameor |
|---|---|---|---|---|---|---|---|---|
| chr2-2 (Ps02_4173 06163) | 1 | scaffold01713:4731 (substituted by chr2:407,448,748) | chr2:396,998,695-413,061,343 | 16,062,649 | (Psat2g170080), Psat2g173360, Psat2g173880 | Psat2g163200-Psat2g173960 | chr2: 82.4 cM | chr2:386,683, 682-424,059,129 |
| chr5-1 (Ps05_ 117545329) | 2 | chr5:78,866,145 | chr5:73,760,833-95,226,690 | 21,465,858 | Psat5g044000 | Psat5g041920-Psat5g052320 | chr5: 31.2 cM | chr5:57,407,9 64-130,197,960 |
| chr5-2 (Ps05_ 604790207) | 3 | chr5:563,627,520 | chr5:556,247,106-578,781,780 | 22,534,675 | Psat5g296560, Psat5g296640 | Psat5g290120-Psat5g308080 | chr5: 130.4 cM | chr5:541,653, 217-(end of chr) |

**Table 2B. Yield QTLs in the GWAS population mapped to the Cameor v1a reference genome**

| QTL | *p*-value peak | *p*-value range | Reference/ variant alleles | SEQ ID NO: | Variant allele frequency | Beta (effect size) per allele | LD block [bp] based on R² = 0.9 cutoff | Pleiotropic effect |
|---|---|---|---|---|---|---|---|---|
| chr2-2 (Ps02_417306 163) | 2.68 x 10¹⁴ | < 1 x 10⁸ | **T/**C | 1 | 0.058 | -9.01 | 9,500,000 | lodging |
| chr5-1 (Ps05_ 117545329) | 3.48 x 10⁸ | < 1 x 10⁷ | **A**/T | 2 | 0.051 | -6.82 | 45,000,000 | |
| chr5-2 (Ps05_ 604790207) | 3.31 x 10¹⁴ | < 1 x 10⁷ | **C/**T | 3 | 0.062 | -9.17 | 5,500,000 | lodging, height |

**Table 3. Effect of yield chr2-2 QTL on yield, height, lodging, and protein content**

| Ps02_417306163_T_C | Mean protein content [%] | Mean yield [bushels/acre] | Mean lodging [units] * | Mean height [inch]** |
|---|---|---|---|---|
| **Homozygous reference alleles (TT)** | 27.11 | **50.81** | 3.18 | 25.98 |
| Heterozygous alleles (TC) | 28.77 | 34.27 | 3.70 | 32.32 |
| Homozygous variant alleles (CC) | 28.74 | 31.17 | 5.11 | 30.94 |
| Difference between homozygous alleles | 1.63 | -19.64 | 1.93 | 4.96 |
| Effect size | | 18.02 | | |

| | | | | |
|---|---|---|---|---|
| * Lodging units are expressed in the scale from 1 to 9, 9 being the worst ** At physiological maturity | | | | |

**Table 4. Effect of yield chr5-1 QTL on yield, height, lodging, and protein content**

| Ps05_117545329_A_T | Mean protein content [%] | Mean yield [bushels/acre] | Mean lodging [units] * | Mean height [inch]** |
|---|---|---|---|---|
| **Homozygous reference alleles (AA)** | 27.13 | **50.64** | 3.19 | 26.06 |
| Heterozygous alleles (AT) | 28.12 | 35.79 | 4.22 | 27.41 |
| Homozygous variant alleles (TT) | 28.84 | 31.18 | 5.11 | 32.44 |
| Difference between homozygous alleles | 1.71 | -19.46 | 1.92 | 6.38 |
| Effect size | | 13.64 | | |

| | | | | |
|---|---|---|---|---|
| * Lodging units are expressed in the scale from 1 to 9, 9 being the worst ** At physiological maturity | | | | |

**Table 5. Effect of yield chr5-2 QTL on yield, height, lodging, and protein content**

| Ps05_604790207_C_T | Mean protein content [%] | Mean yield [bushels/acre] | Mean lodging [units] * | Mean height [inch]** |
|---|---|---|---|---|
| **Homozygous reference alleles (CC)** | 27.13 | 50.77 | 3.17 | 25.98 |
| Heterozygous alleles (CT) | 27.02 | 33.33 | 4.59 | 27.49 |
| Homozygous variant alleles (TT) | 28.79 | 31.53 | 5.12 | 31.97 |
| Difference between homozygous alleles | 1.66 | -19.24 | 1.95 | 5.99 |
| Effect size | | 18.34 | | |

| | | | | |
|---|---|---|---|---|
| * Lodging units are expressed in the scale from 1 to 9, 9 being the worst ** At physiological maturity | | | | |

**Table 6A. Lodging QTLs in the GWAS population mapped to the Cameor v1a reference genome**

| QTL | SEQ ID NO: | Peak SNP Cameor | Peak Range Cameor | Size [bp] Cameor | Peak ID Cameor | ID range Cameor | Genetic Map Position Cameor | ~20 cM range (+/-10 cM) Cameor |
|---|---|---|---|---|---|---|---|---|
| Chr2-1 (Ps02_4157 27951) | 4 | Chr2:409,407,644 | Chr2:399,354,114-413,061,343 | 13,707,230 | Psat2g171320, Psat2g171360 | Psat2g165400-Psat2g173960 | chr2LG1: 83.1 cM | chr2:387,250,594 427,410,060 |
| Chr5-1 (Ps05_6082 56598) | 5 | Chr5:566,655,216 | chr5:542,166,429-572,706,173 | 30,539,744 | Psat5g299360, Psat5g299400 | Psat5g278240-Psat5g303720 | chr5LG3: 132 cM | chr5:547,677,746 (end of chr) |
| Chr6-1 (Ps06_5054 240) | 6 | chr6:6,011,352 | Chr6:2,127,888-8,421,777 | 6,293,890 | Psat6g008280, Psat6g008320 | Psat6g003200-Psat6g011640 | chr6LG2: 2.6 cM | (start of chr)-chr6:17,888,751 |

**Table 6B. Lodging QTLs in the GWAS population mapped to the Cameor v1a reference genome**

| QTL | *p*-value peak, | *p*-value range | Reference /variant alleles | SEQ ID NO: | Variant allele frequency | Beta (effect size) per allele | LD block [bp] based on R² = 0.9 cutoff | Pleiotropic effect |
|---|---|---|---|---|---|---|---|---|
| Chr2-1 (Ps02 _41572 7951) | 1.12 x 10⁻²² | < 1 x 10⁻¹² | C/T | 4 | 0.059 | 9.57 x 10⁻¹ | 17,200,000 | yield, height |
| Chr5-1 (Ps05_60825 6598) | 7.44 x 10⁻²⁴ | < 1 x 10⁻¹² | G/A | 5 | 0.058 | 9.86 x 10⁻¹ | 18.800,000 | yield |
| Chr6-1 (Ps06_50542 40) | 9.76 x 10⁻¹⁶ | < 1 x 10⁻¹² | G/C | 6 | 0.044 | 8.35 x 10⁻¹ | 11,300,00 | height |

**Table 7. Effect of lodging chr2-1 QTL on yield, height, lodging, and protein content**

| Ps02_415727951_C_T | Mean protein content [%] | Mean yield [bushels/acre] | Mean lodging [units] * | Mean height [inch]** |
|---|---|---|---|---|
| **Homozygous reference alleles (CC)** | 27.13 | 50.71 | **3.17** | 26.02 |
| Heterozygous alleles (CT) | 27.54 | 33.47 | 3.93 | 28.63 |
| Homozygous variant alleles (TT) | 28.85 | 31.85 | 5.24 | 31.67 |
| Difference between homozygous alleles | 1.72 | -18.86 | 2.07 | 5.65 |
| Effect size | | | 1.91 | |

| | | | | |
|---|---|---|---|---|
| * Lodging units are expressed in the scale from 1 to 9, 9 being the worst ** At physiological maturity | | | | |

**Table 8. Effect of lodging chr5-1 QTL on yield, height, lodging, and protein content**

| Ps05_608256598_G_A | Mean protein content [%] | Mean yield [bushels/acre] | Mean lodging [units]* | Mean height [inch]** |
|---|---|---|---|---|
| **Homozygous reference alleles (GG)** | 27.12 | 50.76 | **3.17** | 25.97 |
| Heterozygous alleles (GA) | 28.64 | 33.92 | 4.28 | 30.95 |
| Homozygous variant alleles (AA) | 28.67 | 31.43 | 5.26 | 31.83 |
| Difference between homozygous alleles | 1.55 | -19.32 | 2.09 | 5.86 |
| Effect size | | | 1.97 | |

| | | | | |
|---|---|---|---|---|
| * Lodging units are expressed in the scale from 1 to 9, 9 being the worst ** At physiological maturity | | | | |

**Table 9. Effect of lodging chr6-1 QTL on yield, height, lodging, and protein content**

| Ps06_5054240_G_C | Mean protein content [%] | Mean yield [bushels/acre] | Mean lodging [units]* | Mean height [inch]** |
|---|---|---|---|---|
| **Homozygous reference alleles (GG)** | 27.15 | 50.34 | **3.19** | 26.05 |
| Heterozygous alleles (GC) | 29.04 | 33.43 | 4.96 | 39.57 |
| Homozygous variant alleles (CC) | 28.59 | 32.75 | 5.38 | 31.51 |
| Difference between homozygous alleles | 1.45 | -17.59 | 2.18 | 5.45 |
| Effect size | | | 1.67 | |

| | | | | |
|---|---|---|---|---|
| * Lodging units are expressed in the scale from 1 to 9, 9 being the worst ** At physiological maturity | | | | |

**Table 10A. Height QTLs in the GWAS population mapped to the Cameor v1a reference genome**

| QTL | SEQ ID NO: | Peak SNP Cameor | Peak Range Cameor | Size [bp] Cameor | Peak ID Cameor | ID range Cameor | Genetic Map Position Cameor | ~20 cM range (+/-10 cM) Cameor |
|---|---|---|---|---|---|---|---|---|
| Chr2-2 (Ps2_ 390480167) | 7 | ~ chr2LG1:384,481, 633 | chr2:383,969,109-410,578,170 (412,542,086) | 26,609,062 (28,572,97 8) | Psat2g154280 | Psat2gl53920-Psat2gl72480-Psat2g173520 | chr2LG1: 71.1 cM | chr2:354,324,649 -402,078,389 |
| Chr3-1 (Ps03_7415 8043) | 8 | scaffold02911:740 88 (substituted by chr3:35,492,344 (Psat3g016080)) | chr3:26,512,659-42,565,900 | 16,053,242 | Psat3g016080 | Psat3g011240-Psat3g019320 | chr3LG5: 20.5 cM | chr3:18,873,917-69,320,372 |
| Chr3-2 (Ps03_ 496270063) | 9 | chr3LG5:404,873, 882 | chr3:404272358-407100543 | 2,823,186 | Psat3gl89760-Psat3gl89840 | Psat3g189240-Psat3g190840 | chr3LG5: 94.4 cM | chr3:273,327,332 -422,042,733 |
| Chr4-1 (Ps04_ 192115819) | 10 | scaffold03924:74,4 87 (substituted by | chr4:149,505,479-180,857,497 | 31,352,019 | Psat4g088400 | Psat4g085120-Psat4g096640 | chr4LG4: 55 cM | chr4:118,944,413 -200,876,233 |
| | | chr4:158,451,622 (Psat4g088400)) | | | | | | |
| Chr6-1 (Ps06_ 38889673) | 11 | chr6LG2:31,021,5 63 | chr6:2 980 326-52,843,007 | 49,862,682 | Psat6g037840-Psat6g037880 | Psat6g004440-Psat6g053800 | chr6LG2: 22 cM | chr6:17,641,704-56,905,305 |
| Chr7-1 (Ps07_ 115631962) | 12 | chr7LG7:115,361, 106 | chr7:108,733,572-121,361,461 | 12,627,890 | Psat7g070000-Psat7g070080 | Psat7g067000-Psat7g072360 | chr7LG7: 35.9 cM | chr7:79,787,890-153,689,044 |
| Chr7-2 (Ps07_ 129458029) | 13 | chr7LG7:131,513, 318 | chr7:130,685,060-143,521,534 | 12,836,475 | Psat7g078840-Psat7g078960 | Psat7g078560-Psat7g085640 | chr7LG7: 39.2 cM | chr7:91,738,904-165,762,735 |
| Chr7-3 (Ps07_ 256289873) | 14 | scaffold04080:13,8 57 (substituted by chr7:324,031,049 (Psat7g169760)) | chr7:201,578,075-324,633,219 | 123,055,14 5 | Psat7g169760 | Psat7g122160-Psat7g169960 | chr7LG7: 68.8 cM | chr7:207,998,578 -360,550,624 |

**Table 10B. Height QTLs in the GWAS population mapped to the Cameor v1a reference genome**

| QTL | SEQ ID NO: | *p*-value peak | *p*-value range | Reference /variant alleles | Variant allele frequency | Beta (effect size) per allele | LD block [bp] based on R² = 0.9 cutoff | Pleiotropic effect |
|---|---|---|---|---|---|---|---|---|
| Chr2-2 (Ps2_ 390480167) | 7 | 3.16 x 10⁻¹⁰ | < 1 x 10⁻⁷ | T**/C** | 0.026 | 4.97E+00 | 3,000,000 | yield, lodging |
| Chr3-1 (Ps03_741580 43) | 8 | 6.63 x 10⁻¹¹ | < 1 x 10⁻⁷ | A**/G** | 0.03 | 4.67E+00 | 3,800,000 (9,200,000) | NA |
| Chr3-2 (Ps03_ 496270063) | 9 | 8.89 x 10⁻¹⁰ | < 1 x 10⁻⁷ | G**/A** | 0.031 | 4.46E+00 | 92 | NA |
| Chr4-1 (Ps04_ 192115819) | 10 | 1.56 x 10⁻¹³ | < 1 x 10⁻⁷ | C/**T** | 0.025 | 5.41E+00 | 39,800,000 | lodging |
| Chr6-1 (Ps06_ 38889673) | 11 | 2.70 x 10⁻¹³ | < 1 x 10⁻⁷ | C/**T** | 0.022 | 5.47E+00 | 7,000,000 | lodging |
| Chr7-1 (Ps07_ 115631962) | 12 | 4.77 x 10⁻¹⁷ | < 1 x 10⁻¹³ | G/**A** | 0.026 | 6.39E+00 | >65,000,000 (9,200,000) | NA |
| Chr7-2 (Ps07_ 129458029) | 13 | 3.72 x 10 - 16 | < 1 x 10⁻¹³ | A/**G** | 0.037 | 5.09E+00 | >65,000,000 | NA |
| Chr7-3 (Ps07_ 256289873) | 14 | 2.25 x 10 - 15 | < 1 x 10⁻¹¹ | G/**A** | 0.023 | 6.62E+00 | 17,000,000 | NA |

**Table 11. Effect of height chr2-2 QTL on yield, height, lodging, and protein content**

| Ps02_390480167_T_C | Mean protein content [%] | Mean yield [bushels/acre] | Mean lodging [units]* | Mean height [inch]** |
|---|---|---|---|---|
| Homozygous reference alleles (TT) | 27.16 | 50.22 | 3.23 | 25.99 |
| Heterozygous alleles (TC) | 28.58 | 29.00 | 4.98 | 33.86 |
| **Homozygous variant alleles (CC)** | 28.94 | 34.67 | 5.00 | 36.25 |
| Difference between homozygous alleles | 1.78 | -15.55 | 1.77 | 10.26 |
| Effect size | | | | 9.94 |

| | | | | |
|---|---|---|---|---|
| * Lodging units are expressed in the scale from 1 to 9, 9 being the worst ** At physiological maturity | | | | |

**Table 12. Effect of height chr3-1 QTL on yield, height, lodging, and protein content**

| Ps03_74158043_A_G | Mean protein content [%] | Mean yield [bushels/acre] | Mean lodging [units]* | Mean height [inch]** |
|---|---|---|---|---|
| Homozygous reference alleles (AA) | 27.15 | 50.22 | 3.23 | 26.03 |
| Heterozygous alleles (AG) | 28.80 | 30.70 | 5.01 | 30.61 |
| **Homozygous variant alleles (GG)** | 29.07 | 33.82 | 4.89 | **35.84** |
| Difference between homozygous alleles | 1.92 | -16.39 | 1.67 | 9.81 |
| Effect size | | | | 9.34 |

| | | | | |
|---|---|---|---|---|
| * Lodging units are expressed in the scale from 1 to 9, 9 being the worst ** At physiological maturity | | | | |

**Table 13. Effect of height chr3-2 QTL on yield, height, lodging, and protein content**

| Ps03_496270063_G_A | Mean protein content [%] | Mean yield [bushels/acre] | Mean lodging [units]* | Mean height [inch]** |
|---|---|---|---|---|
| Homozygous reference alleles (GG) | 27.18 | 50.05 | 3.23 | 26.02 |
| Heterozygous alleles (GA) | 27.13 | 43.21 | 4.46 | 31.56 |
| **Homozygous variant alleles (AA)** | 28.73 | 32.95 | 4.99 | **35.30** |
| Difference between homozygous alleles | 1.55 | -17.10 | 1.76 | 9.28 |
| Effect size | | | | 8.92 |

| | | | | |
|---|---|---|---|---|
| * Lodging units are expressed in the scale from 1 to 9, 9 being the worst ** At physiological maturity | | | | |

**Table 14. Effect of height chr4-1 QTL on yield, height, lodging, and protein content**

| Ps04_192115819_C_T | Mean protein content [%] | Mean yield [bushels/acre] | Mean lodging [units]* | Mean height [inch]** |
|---|---|---|---|---|
| Homozygous reference alleles (CC) | 27.16 | 50.08 | 3.23 | 26.01 |
| Heterozygous alleles (CT) | 28.70 | 35.71 | 5.10 | 32.27 |
| **Homozygous variant alleles (TT)** | 29.06 | 32.22 | 5.19 | **37.93** |
| Difference between homozygous alleles | 1.90 | -17.86 | 1.96 | 11.92 |
| Effect size | | | | 10.82 |

| | | | | |
|---|---|---|---|---|
| * Lodging units are expressed in the scale from 1 to 9, 9 being the worst ** At physiological maturity | | | | |

**Table 15. Effect of height chr6-1 QTL on yield, height, lodging, and protein content**

| Ps06_38889673_C_T | Mean protein content [%] | Mean yield [bushels/acre] | Mean lodging [units] * | Mean height [inch]** |
|---|---|---|---|---|
| Homozygous reference alleles (CC) | 27.18 | 49.98 | 3.25 | 26.04 |
| Heterozygous alleles (CT) | 27.68 | 37.18 | 3.40 | 29.29 |
| **Homozygous variant alleles (TT)** | 28.79 | 33.48 | 5.31 | **39.13** |
| Difference between homozygous alleles | 1.61 | -16.50 | 2.07 | 13.09 |
| Effect size | | | | 10.94 |

| | | | | |
|---|---|---|---|---|
| * Lodging units are expressed in the scale from 1 to 9, 9 being the worst ** At physiological maturity | | | | |

**Table 16. Effect of height chr7-1 QTL on yield, height, lodging, and protein content**

| Ps07_115631962_G_A | Mean protein content [%] | Mean yield [bushels/acre] | Mean lodging [units] * | Mean height [inch]** |
|---|---|---|---|---|
| Homozygous reference alleles (GG) | 27.15 | 50.21 | 3.23 | 25.98 |
| Heterozygous alleles (GA) | 29.15 | 31.06 | 4.99 | 31.43 |
| **Homozygous variant alleles (AA)** | 28.80 | 32.80 | 4.87 | **39.63** |
| Difference between homozygous alleles | 1.64 | -17.40 | 1.65 | 13.65 |
| Effect size | | | | 12.78 |

| | | | | |
|---|---|---|---|---|
| * Lodging units are expressed in the scale from 1 to 9, 9 being the worst ** At physiological maturity | | | | |

**Table 17. Effect of height chr7-2 QTL on yield, height, lodging, and protein content**

| Ps07_129458029_A_G | Mean protein content [%] | Mean yield [bushels/acre] | Mean lodging [units] * | Mean height [inch]** |
|---|---|---|---|---|
| Homozygous reference alleles (AA) | 27.14 | 50.31 | 3.22 | 25.96 |
| Heterozygous alleles (AG) | 28.55 | 37.83 | 4.42 | 30.14 |
| **Homozygous variant alleles (GG)** | 29.04 | 31.92 | 4.91 | **36.52** |
| Difference between homozygous alleles | 1.90 | -18.39 | 1.69 | 10.56 |
| Effect size | | | | 10.18 |

| | | | | |
|---|---|---|---|---|
| * Lodging units are expressed in the scale from 1 to 9, 9 being the worst ** At physiological maturity | | | | |

**Table 18. Effect of height chr7-3 QTL on yield, height, lodging, and protein content**

| Ps07_256289873_G_A | Mean protein content [%] | Mean yield [bushels/acre] | Mean lodging [units] * | Mean height [inch]** |
|---|---|---|---|---|
| Homozygous reference alleles (GG) | 27.16 | 50.16 | 3.23 | 26.02 |
| Heterozygous alleles (GA) | 28.31 | 32.82 | 4.54 | 32.31 |
| **Homozygous variant alleles (AA)** | 29.32 | 33.91 | 5.21 | **39.64** |
| Difference between homozygous alleles | 2.16 | -16.25 | 1.98 | 13.62 |
| Effect size | | | | 13.24 |

| | | | | |
|---|---|---|---|---|
| * Lodging units are expressed in the scale from 1 to 9, 9 being the worst ** At physiological maturity | | | | |

### Example 2. Identifying single nucleotide polymorphisms (SNPs) for least absolute shrinkage and selection operator (LASSO) model based genomic selection in yield, height, and lodging phenotypes

SNPs relevant to genomic selection based on the in-house reference genome in yield, height, and lodging phenotypes were identified using least absolute shrinkage and selection operator (LASSO) genomic prediction model coefficient 99^{th} percentile cutoff. The genetic map position and public physical position of the SNPs in the Cameor v1a reference genome ("Cameor") were obtained using BLAST. An approximated public physical position was obtained in cases where the SNPs were mapped to scaffold or incorrect chromosome in Cameor. Sequences 100 bp upstream and downstream of the SNP were also obtained.

The identified markers are presented in Table 19. Each marker identified in Table 19 includes the SNP marker with 100 nucleotide example/representative genomic sequences upstream and downstream of the SNP marker. The sequence upstream and downstream of the SNP may vary. Each marker (such as "Ps01_12111263") provided herein comprises the SNP marker indicated in Table 19, with upstream and downstream nucleic acid sequences that have at least 85% sequence identity with the nucleic acid sequence indicated in Table 19 for each marker. Each marker listed in Table 19 (referred to as "marker AA") can also refer to an SNP marker at position 101 of the nucleic acid sequence identified for the marker AA (referred to as "SEQ ID NO: BB") in a genomic region comprising the nucleic acid sequence of SEQ ID NO: BB, or at a corresponding position of a genomic region at least about 50, 100, 150, or 200 nucleotides of which is aligned to SEQ ID NO: BB for maximum homology (e.g., 85%, 86%, 87%, 88%, 89%, 90%, 91%, 92%, 93%, 94%, 95%, 96%, 97%, 98%, or 99% homology) when using standard alignment parameters. For example, the SNP marker identified as Ps01_12111263 can have a sequence that has at least 85% identity with SEQ ID NO: 15. The marker identified as Ps01_12111263 can also refer to an SNP marker at position 101 of a nucleic acid sequence of SEQ ID NO: 15 in a genomic region comprising the nucleic acid sequence of SEQ ID NO: 15, or at a corresponding position of a genomic region at least about 50, 100, 150, or 200 nucleotides of which is aligned to SEQ ID NO: 15 for maximum homology (e.g., 85%, 86%, 87%, 88%, 89%, 90%, 91%, 92%, 93%, 94%, 95%, 96%, 97%, 98%, or 99% homology) when using standard alignment parameters.

Yield LASSO coefficient, height LASSO coefficient, and lodging LASSO coefficient represent strength of association between the marker and increased yield, increased height, and decreased lodging, respectively, with a higher absolute value indicating higher association between the marker and the phenotype. A positive LASSO coefficient indicates that the variant allele of the marker is associated with the phenotype (i.e., high yield, high height, high lodging). A negative LASSO coefficient indicates that the reference allele of the marker is associated with the phenotype (i.e., high yield, high height, high lodging). A typical desirable phenotype is high yield and low lodging. In addition, a high height or low height phenotype can be desirable depending on other phenotypes and circumstances. For example, between two plants having similar yield, low height may be a desirable phenotype because lower plants are less likely to lodge. Therefore, a desirable allele can have positive yield LASSO coefficient, and/or negative lodging LASSO coefficient, optionally in combination with positive or negative height LASSO coefficient depending on the situation.

Yield, lodging, and height are correlated phenotypes. For example, Table 20 lists 4 pleiotropic LASSO markers (among those identified in Table 19) that are significantly associated with more than one phenotype.

Markers identified in the GWAS population and in the LASSO model can overlap. For example, Table 21 lists 17 LASSO markers (among those identified in Table 19) that are also within the marker peak ranges identified in the GWAS population in Tables 3, 6, and 10. Among the 17 overlapping markers, 9 are within the GWAS peaks of the same phenotype as the LASSO marker phenotype.

For example, marker Ps02_406013484 (allele C) is associated with high yield and low lodging based on the LASSO model, and overlaps with the yield and height chr2-2 GWAS peak associated with high yield and high height.

**Table 19. SNP markers associated with yield, height, and/or lodging phenotypes in pea**

| Marker | SNP alleles (reference /variant) | SEQ ID NO: | Cameor v1a chromosome | Cameor v1a physical position | Approxi mated Cameor position | LASSO Coefficient | | | Significant Phenotype | Overlap with GWAS markers |
|---|---|---|---|---|---|---|---|---|---|---|
| | | | | | | Yield | Lodging | Height | | |
| Ps01_12111263 | T/G | 15 | chr4LG4 | 128561964 | 17194777 | -0.287 | 0 | 0 | Yield | |
| Ps01_21627814 | T/C | 16 | scaffold00011 | 4366 | 24551584 | -0.446 | 0 | 0 | Yield | |
| Ps01_80920094 | C/T | 17 | chr1LG6 | 73778015 | | 0.340 | 0 | 0 | Yield | |
| Ps01_165828422 | G/A | 18 | chr1LG6 | 150246299 | | -0.560 | 0 | 0 | Yield | |
| Ps01_172888276 | G/T | 19 | chr1LG6 | 151466935 | | -0.328 | 0 | 0 | Yield | |
| Ps01_274311344 | G/A | 20 | chr1LG6 | 214738954 | | 1.200 | 0 | 0 | Yield | |
| Ps02_277385615 | C/T | 21 | chr2LG1 | 287196406 | | 0.537 | 0 | 0 | Yield | |
| Ps01_306049485 | A/G | 22 | scaffold05541 | 12681 | 24442486 6 | 0.348 | 0 | 0 | Yield | |
| Ps01_345383217 | C/T | 23 | chr1LG6 | 290859608 | | -0.303 | 0 | 0 | Yield | |
| Ps01_414516587 | A/C | 24 | chr1LG6 | 347862320 | | 0.398 | 0 | 0 | Yield | |
| Ps02_389051201 | G/A | 25 | chr2LG1 | 383268719 | | -0.367 | 0 | 0.020 | Yield | |
| Ps02_406013484 | T/C | 26 | chr2LG1 | 397000005 | | 0.390 | -0.098 | 0 | Yield Lodging | Yield (Chr2-2), Height (Chr2-2) |
| Ps02_415945622 | G/A | 27 | chr2LG1 | 405820442 | | -0.387 | 0.011 | 0 | Yield | Yield (Chr2-2), Height (Chr2-2), Lodging (Chr2-1) |
| Ps03_98286373 | G/A | 28 | chr3LG5 | 55052537 | | 0.369 | 0 | 0 | Yield | |
| Ps04_33913844 | A/T | 29 | chr4LG4 | 22316149 | | 0.294 | 0 | 0 | Yield | |
| Ps04_383816669 | G/T | 30 | chr4LG4 | 398264448 | | -0.455 | 0 | 0 | Yield | |
| Ps04_445153308 | C/A | 31 | chr4LG4 | 374998060 | | -0.491 | 0.162 | 0 | Yield_ Lodging | |
| Ps05_48702172 | A/G | 32 | chr5LG3 | 35018291 | | -0.304 | 0 | -0.092 | Yield | |
| Ps05_237258346 | A/G | 33 | chr5LG3 | 190837200 | | 0.483 | 0 | 0 | Yield | |
| Ps05_318938718 | A/G | 34 | chr5LG3 | 12480905 | | 0.290 | 0 | -0.022 | Yield | |
| Ps05_320108884 | T/C | 35 | chr5LG3 | 268153146 | | -0.312 | 0 | 0 | Yield | |
| Ps05_526760209 | A/G | 36 | chr5LG3 | 489005341 | | -0.587 | 0.003 | 0 | Yield | |
| Ps05_536892706 | T/C | 37 | chr5LG3 | 496475925 | | -0.312 | 0 | 0 | Yield | |
| Ps06_32259152 | A/G | 38 | scaffold00839 | 4102 | 12702042 | -0.285 | 0 | -0.083 | Yield | Height (Chr6-1) |
| Ps06_43753843 | A/G | 39 | chr6LG2 | 332130985 | | 0.343 | 0 | 0 | Yield | Height (Chr6-1) |
| Ps06_105337899 | C/T | 40 | chr6LG2 | 56896622 | | 0.596 | 0 | 0 | Yield | |
| Ps06_127479778 | G/A | 41 | chr6LG2 | 112269847 | | 0.666 | 0 | 0 | Yield | |
| Ps06_367630606 | C/T | 42 | chr6LG2 | 368401417 | | -0.283 | 0 | 0 | Yield | |
| Ps06_427519500 | C/G | 43 | chr6LG2 | 426328693 | | 0.601 | 0 | 0 | Yield | |
| Ps07_9801763 | G/A | 44 | chr7LG7 | 8316115 | | 0.488 | -0.339 | 0 | Yield Lodging | |
| Ps07_39866790 | T/C | 45 | chr7LG7 | 37445789 | | -0.395 | 0 | 0 | Yield | |
| Ps07_129103458 | G/A | 46 | chr7LG7 | 132484304 | | 0.318 | 0 | 0 | Yield | Height (Chr7-2) |
| Ps07_142955054 | G/A | 47 | chr7LG7 | 144420546 | | 0.331 | 0 | 0 | Yield | |
| Ps07_173064631 | C/T | 48 | chr7LG7 | 172275819 | | -0.454 | 0 | 0 | Yield | |
| Ps07_173321635 | G/A | 49 | chr7LG7 | 16557144 | | -0.577 | 0 | 0 | Yield | |
| Ps07_278403545 | T/C | 50 | scaffold02229 | 22321 | 28529960 0 | 0.573 | 0 | 0 | Yield | |
| Ps07_303005109 | G/A | 51 | chr7LG7 | 359039170 | | -0.304 | 0.009 | 0 | Yield | |
| Ps07_481516842 | A/T | 52 | chr7LG7 | 481251260 | | 0.300 | 0 | 0 | Yield | |
| Ps01_279286967 | A/G | 53 | chr1LG6 | 122338217 | | -0.136 | 0.107 | 0 | Lodging | |
| Ps01_405953972 | T/C | 54 | chr1LG6 | 340372022 | | 0 | -0.177 | 0 | Lodging | |
| Ps02_66314510 | T/C | 55 | chr2LG1 | 41766109 | | 0 | 0.068 | 0 | Lodging | |
| Ps02_146578429 | G/A | 56 | chr2LG1 | 12117951 | | 0 | 0.068 | 0 | Lodging | |
| Ps03_114813976 | A/C | 57 | chr3LG5 | 67204106 | | 0 | 0.050 | 0 | Lodging | |
| Ps03_120645295 | A/C | 58 | scaffold06018 | 3352 | 73696006 | 0 | -0.103 | 0.045 | Lodging | |
| Ps03_230771576 | C/T | 59 | chr3LG5 | 166350011 | | -0.102 | 0.054 | 0 | Lodging | |
| Ps03_253149185 | C/T | 60 | scaffold03592 | 85406 | 18737872 6 | 0 | 0.060 | 0 | Lodging | |
| Ps03_512230003 | G/T | 61 | chr3LG5 | 420398638 | | -0.039 | 0.067 | 0 | Lodging | |
| Ps03_531239107 | T/C | 62 | chr5LG3 | 563531892 | 42596808 8 | 0 | -0.067 | 0.250 | Lodging_ Height | |
| Ps03_71303964 | G/A | 63 | super-scaffold8606 | 249079 | 31363236 | 0 | 0.051 | 0 | Lodging | Height (Chr3-1) |
| Ps04_69656387 | T/A | 64 | chr4LG4 | 47145729 | | 0 | -0.318 | 0 | Lodging | |
| Ps04_144177304 | T/C | 65 | chr4LG4 | 119133416 | | 0 | -0.079 | 0 | Lodging | |
| Ps04_156326296 | G/A | 66 | chr4LG4 | 130758952 | | 0 | 0.035 | 0 | Lodging | |
| Ps04_260958274 | C/T | 67 | scaffold02661 | 83020 | 22887765 2 | 0 | 0.066 | 0 | Lodging | |
| Ps04_264937409 | C/T | 68 | chr3LG5 | 403189011 | 23258590 4 | 0 | 0.455 | 0 | Lodging | |
| Ps04_374148702 | T/C | 69 | chr4LG4 | 285109838 | | 0.041 | 0.047 | 0 | Lodging | |
| Ps04_453044385 | C/T | 70 | chr4LG4 | 440853776 | | 0 | -0.088 | 0 | Lodging | |
| Ps05_3468595 | G/A | 71 | chr5LG3 | 3579419 | | 0 | -0.117 | 0 | Lodging | |
| Ps05_223782536 | T/A | 72 | chr5LG3 | 187232313 | | 0 | -0.181 | 0 | Lodging | |
| Ps05_352490739 | C/A | 73 | chr5LG3 | 84459119 | | 0 | -0.066 | 0 | Lodging | |
| Ps05_473638215 | T/C | 74 | chr5LG3 | 438580908 | | 0 | -0.123 | 0 | Lodging | |
| Ps05_481936646 | T/A | 75 | super-scaffold9678 | 10598 | 43771116 0 | 0 | 0.051 | 0 | Lodging | |
| Ps05_528041290 | A/G | 76 | chr5LG3 | 489523514 | | 0 | -0.092 | 0 | Lodging | |
| Ps05_551582581 | G/A | 77 | chr5LG3 | 509926470 | | 0 | 0.057 | 0 | Lodging | |
| Ps05_583707944 | C/T | 78 | chr5LG3 | 543282804 | | 0 | -0.066 | 0 | Lodging | Lodging (Chr5-1) |
| Ps05_612824899 | C/T | 79 | chr5LG3 | 570360377 | | 0.232 | -0.088 | 0 | Lodging | Yield (Chr5-2), Lodging (Chr5-1) |
| Ps05_616716582 | T/C | 80 | chr5LG3 | 8937964 | | 0 | -0.087 | 0 | Lodging | Yield (Chr5-2) |
| Ps06_12615016 | G/A | 81 | chr6LG2 | 11907302 | | 0.140 | -0.143 | 0 | Lodging | Height (Chr6-1) |
| Ps06_33377536 | T/A | 82 | chr6LG2 | 2560210 | | 0 | -0.068 | 0 | Lodging | Height (Chr6-1) |
| Ps06_349137016 | T/G | 83 | chr6LG2 | 348314640 | | 0 | -0.090 | 0 | Lodging | |
| Ps06_425993896 | C/A | 84 | chr6LG2 | 185310300 | | -0.018 | 0.079 | 0 | Lodging | |
| Ps07_133823647 | G/A | 85 | chr2LG1 | 112005091 | 13760070 8 | 0 | 0.039 | 0 | Lodging | Height (Chr7-2) |
| Ps07_194142598 | A/G | 86 | chr7LG7 | 191955823 | | 0 | 0.115 | 0 | Lodging | |
| Ps07 195412515 | G/A | 87 | chr7LG7 | 195183596 | | 0 | 0.076 | 0.008 | Lodging | |
| Ps01_68070331 | T/A | 88 | chr1LG6 | 63453702 | | 0 | 0 | 0.146 | Height | |
| Ps02_233036770 | T/C | 89 | chr2LG1 | 96628783 | | 0 | 0 | -0.347 | Height | |
| Ps02_342684459 | C/T | 90 | chr2LG1 | 341987428 | | 0 | 0 | -0.239 | Height | |
| Ps02_426672885 | C/G | 91 | chr2LG1 | 415226641 | | 0 | 0 | -0.154 | Height | |
| Ps02_433458109 | C/T | 92 | scaffold05147 | 6226 | 42530513 8 | 0 | 0 | -0.145 | Height | |
| Ps03_94359486 | T/C | 93 | chr3LG5 | 52540141 | | 0 | 0 | 0.281 | Height | |
| Ps03_104956337 | A/G | 94 | chr7LG7 | 63397608 | 60235545 | 0 | 0 | 0.109 | Height | |
| Ps03_118892995 | A/T | 95 | chr4LG4 | 403366757 | 72192607 | 0 | 0 | -0.185 | Height | |
| Ps03_195398925 | T/G | 96 | chr3LG5 | 137302656 | | -0.097 | 0 | -0.251 | Height | |
| Ps03_205809630 | C/A | 97 | scaffold00254 | 82633 | 14676339 5 | 0 | 0 | 0.283 | Height | |
| Ps03_241957626 | A/T | 98 | chr3LG5 | 175749690 | | 0 | 0 | 0.189 | Height | |
| Ps03_242777502 | C/T | 99 | chr3LG5 | 811842 | | -0.086 | 0 | -0.192 | Height | |
| Ps03_251323511 | A/G | 100 | chr3LG5 | 182785714 | | 0 | 0 | -0.232 | Height | |
| Ps03_374110266 | A/G | 101 | chr3LG5 | 289130533 | | 0 | 0 | 0.125 | Height | |
| Ps03_437895589 | T/C | 102 | chr3LG5 | 294741093 | | 0 | 0 | 0.093 | Height | |
| Ps03_493092288 | G/C | 103 | scaffold03249 | 112747 | 39323973 4 | 0 | 0 | 0.097 | Height | |
| Ps04_205866569 | T/G | 104 | chr4LG4 | 173468835 | | 0 | 0 | -0.365 | Height | Height (Chr4-1) |
| Ps04_389245228 | G/A | 105 | chr4LG4 | 402922728 | | -0.154 | 0 | -0.152 | Height | |
| Ps04_430139407 | C/T | 106 | chr4LG4 | 435276132 | | 0 | 0 | 0.101 | Height | |
| Ps04_448803743 | A/G | 107 | chr4LG4 | 440508849 | | 0 | 0 | 0.116 | Height | |
| Ps05_5262178 | T/C | 108 | chr5LG3 | 362378 | | 0 | 0 | 0.164 | Height | |
| Ps05_160013078 | A/G | 109 | chr5LG3 | 122579305 | | 0 | 0 | -0.333 | Height | |
| Ps05_174010831 | C/A | 110 | scaffold01526 | 123477 | 14223875 8 | 0 | 0 | 0.286 | Height | |
| Ps05_277684648 | A/T | 111 | chr5LG3 | 229071963 | | 0 | 0 | 0.187 | Height | |
| Ps06_213382570 | G/T | 112 | chr6LG2 | 157000374 | | -0.067 | 0 | -0.215 | Height | |
| Ps06_236678213 | A/G | 113 | chr6LG2 | 169305572 | | 0 | 0 | 0.090 | Height | |
| Ps06_398588276 | G/A | 114 | chr6LG2 | 396152218 | | 0 | -0.062 | -0.186 | Height | |
| Ps06_472058597 | C/T | 115 | chr6LG2 | 469077464 | | 0 | 0 | 0.127 | Height | |
| Ps06_478057404 | C/T | 116 | scaffold01649 | 134790 | 47143379 3 | -0.213 | 0 | -0.221 | Height | |
| Ps07_58281807 | A/G | 117 | chr7LG7 | 56384258 | | 0 | 0 | -0.313 | Height | |
| Ps07_105730908 | G/C | 118 | chr7LG7 | 104698039 | | 0 | 0 | -0.286 | Height | |
| Ps07_112377551 | G/A | 119 | chr7LG7 | 45871371 | | 0 | 0.024 | 0.240 | Height | Height (Chr7-1) |
| Ps07_114916793 | G/A | 120 | chr7LG7 | 116467927 | | 0 | 0 | 2.227 | Height | Height (Chr7-1) |
| Ps07_213648906 | A/G | 121 | chr7LG7 | 750954 | | -0.044 | 0 | 0.225 | Height | |
| Ps07_234681662 | C/T | 122 | chr7LG7 | 293412012 | | 0 | 0 | -0.319 | Height | Height (Chr7-3) |
| Ps07_238767894 | T/G | 123 | chr7LG7 | 310516174 | | 0 | 0 | -0.179 | Height | Height (Chr7-3) |
| Ps07_489306364 | A/G | 124 | chr7LG7 | 487220871 | | -0.249 | 0 | -0.159 | Height | |

**Table 20. Pleiotropic Lasso Markers**

| Marker | SNP alleles (reference/ variant) | SEQ ID NO: | Cameor v1a chromosome | Cameor v1a physical position | Approxi mated Cameor position | LASSO Coefficient | | | Significant Phenotype | Overlap with GWAS markers |
|---|---|---|---|---|---|---|---|---|---|---|
| | | | | | | Yield | Lodging | Height | | |
| Ps02_406013484 | T/C | 26 | chr2LG1 | 397000005 | | 0.390 | -0.098 | 0 | Yield Lodging | Yield (Chr2-2), Height (Chr2-2) |
| Ps03_531239107 | T/C | 60 | chr5LG3 | 563531892 | 42596808 8 | 0 | -0.067 | 0.250 | Lodging_ Height | |
| Ps04_445153308 | C/A | 31 | chr4LG4 | 374998060 | | -0.490 | 0.162 | 0 | Yield Lodging | |
| Ps07_9801763 | G/A | 44 | chr7LG7 | 8316115 | | 0.488 | -0.339 | 0 | Yield Lodging | |

**Table 21. GWAS and Lasso overlapping markers**

| Marker | SNP alleles (reference /variant) | SEQ ID NO: | Cameor v1a chromosome | Cameor v1a physical position | Approxi mated Cameor position | LASSO Coefficient | | | Significant Phenotype | Overlap with GWAS markers |
|---|---|---|---|---|---|---|---|---|---|---|
| | | | | | | Yield | Lodging | Height | | |
| Ps02_406013484 | T/C | 26 | chr2LG1 | 397000005 | | 0.390 | -0.098 | 0 | Yield Lodging | Yield (Chr2-2), Height (Chr2-2) |
| Ps02_415945622 | G/A | 27 | chr2LG1 | 405820442 | | -0.387 | 0.011 | 0 | Yield | Yield (Chr2-2), Height (Chr2-2), Lodging (Chr2-1) |
| Ps06_32259152 | A/G | 38 | scaffold00839 | 4102 | 12702042 | -0.285 | 0 | -0.083 | Yield | Height (Chr6-1) |
| Ps06_43753843 | A/G | 39 | chr6LG2 | 332130985 | | 0.343 | 0 | 0 | Yield | Height (Chr6-1) |
| Ps07_129103458 | G/A | 46 | chr7LG7 | 132484304 | | 0.318 | 0 | 0 | Yield | Height (Chr7-2) |
| Ps03_71303964 | G/A | 63 | super-scaffold8606 | 249079 | 31363236 | 0 | 0.051 | 0 | Lodging | Height (Chr3-1) |
| Ps05_583707944 | C/T | 78 | chr5LG3 | 543282804 | | 0 | -0.066 | 0 | Lodging | Lodging (Chr5-1) |
| Ps05_612824899 | C/T | 79 | chr5LG3 | 570360377 | | 0.232 | -0.088 | 0 | Lodging | Yield (Chr5-2), Lodging (Chr5-1) |
| Ps05_616716582 | T/C | 80 | chr5LG3 | 8937964 | | 0 | -0.087 | 0 | Lodging | Yield (Chr5-2) |
| Ps06_12615016 | G/A | 81 | chr6LG2 | 11907302 | | 0.140 | -0.143 | 0 | Lodging | Height (Chr6-1) |
| Ps06_33377536 | T/A | 82 | chr6LG2 | 2560210 | | 0 | -0.068 | 0 | Lodging | Height (Chr6-1) |
| Ps07_133823647 | G/A | 85 | chr2LG1 | 112005091 | 13760070 8 | 0 | 0.039 | 0 | Lodging | Height (Chr7-2) |
| Ps04_205866569 | T/G | 104 | chr4LG4 | 173468835 | | 0 | 0 | -0.365 | Height | Height (Chr4-1) |
| Ps07_112377551 | G/A | 119 | chr7LG7 | 45871371 | | 0 | 0.024 | 0.240 | Height | Height (Chr7-1) |
| Ps07_114916793 | G/A | 120 | chr7LG7 | 116467927 | | 0 | 0 | 2.227 | Height | Height (Chr7-1) |
| Ps07_234681662 | C/T | 122 | chr7LG7 | 293412012 | | 0 | 0 | -0.319 | Height | Height (Chr7-3) |
| Ps07_238767894 | T/G | 123 | chr7LG7 | 310516174 | | 0 | 0 | -0.179 | Height | Height (Chr7-3) |

## Claims

1. A method of producing a population of pea plants or seeds with a desirable yield, height, and/or lodging phenotype, said method comprising:
(a) genotyping a first population of pea plants or seeds for the presence of at least one yield, height, and/or lodging molecular marker that is within 20 centimorgans of one or more yield, height, and/or lodging Quantitative Trait Locus (QTLs) selected from the group consisting of yield chr2-2 (Ps02_417306163), yield chr5-1 (Ps05_117545329), yield chr5-2 (Ps05_604790207), lodging chr2-1 (Ps02_415727951), lodging chr5-1 (Ps05_608256598), lodging chr6-1 (Ps06_5054240), height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364;
(b) selecting from the first population one or more pea plants or seeds comprising one or more yield, height, and/or lodging alleles having the at least one yield, height, and/or lodging molecular marker; and
(c) producing a second population of progeny pea plants or seeds from the selected one or more pea plants or plants grown from the selected seeds, wherein the second population of progeny pea plants or seeds comprises the one or more yield, height, and/or lodging alleles having the at least one yield, height, and/or lodging molecular marker,
and wherein the second population of progeny pea plants or seeds are pea plants or seeds with a desirable yield, height, and/or lodging phenotype, thereby producing a population of pea plants or seeds with a desirable yield, height, and/or lodging phenotype.

2. The method of claim 1, wherein said at least one yield, height, and/or lodging molecular marker is within 10 centimorgans of said one or more yield, height, and/or lodging QTLs.

3. The method of claim 1 or 2, wherein genotyping comprises assaying a single nucleotide polymorphism (SNP) marker, detecting a haplotype, and/or the use of an oligonucleotide probe or a pair of primers.

4. The method of any one of claims 1-3, wherein the population of pea plants or seeds comprising said one or more yield, height, and/or lodging alleles (i) has average yield that is greater by at least 13 bushels per acre, average height that is greater by at least 8 inches, and/or average lodging that is lower by at least 1.5 lodging units relative to a control population of pea plants or seeds without said one or more yield, height, and/or lodging alleles, and/or (ii) has average protein and/or average oil content that is at least 90% relative to a control population of pea plants or seeds without said one or more yield, height, and/or lodging alleles.

5. The population of pea plants of any one of claims 1-4, wherein said second population of pea plants has a greater frequency of the at least one yield, height, and/or lodging molecular marker, and greater yield, greater height, and/or less lodging relative to said first population of pea plants.

6. A method of introgressing a yield, height, and/or lodging QTL, the method comprising:
(a) crossing a first pea plant comprising a yield, height, and/or lodging QTL with a second pea plant of a different genotype to produce one or more progeny plants or seeds; and
(b) selecting a progeny plant or seed comprising one or more yield, height, and/or lodging alleles of a polymorphic locus linked to the yield, height, and/or lodging QTL,
wherein the polymorphic locus corresponds to a chromosomal segment comprising any marker within a genomic region between positions: 386683682-424059129 of chromosome 2; 57407964-130197960 of chromosome 5; 541653217-end of chromosome 5; 387250594-427410060 of chromosome 2; 547677746-end of chromosome 5; start-17888751 of chromosome 6; 354324649-402078389 of chromosome 2; 18873917-69320372 of chromosome 3; 273327332-422042733 of chromosome 3; 118944413-200876233 of chromosome 4; 17641704-56905305 of chromosome 6; 79787890-153689044 of chromosome 7; 91738904-165762735 of chromosome 7; 207998578-360550624 of chromosome 7; scaffold00011; 73778015-347862320 of chromosome 1 LG6; 287196406-405820442 of chromosome 2 LG1; scaffold05541; 22316149-374998060 of chromosome 4 LG4; 35018291-496475925 of chromosome 5 LG3; 4102 if scaffold00839; 332130985-426328693 of chromosome 6 LG2; 8316115-481251260 of chromosome 7 LG7; scaffold02229; 122338217-340372022 of chromosome 1 LG6; 12117951-41766109 of chromosome 2 LG1; 55052537-420398638 of chromosome 3 LG5; scaffold06018; scaffold03592; super-scaffold8606; 47145729-440853776 of chromosome 4 LG4; scaffold02661; 3579419-570360377 of chromosome 5LG3; super-scaffold9678; 2560210-348314640 of chromosome 6 LG2; 191955823-63397608 of chromosome 7 LG7; 63453702 of chromosome 1 LG6; 96628783-415226641 of chromosome 2 LG1; scaffold05147; 403366757 of chromosome 4 LG4; scaffold00254; 811842-294741093 of chromosome 3 LG5; scaffold03249; 173468835-440508849 of chromosome 4 LG4; 362378-229071963 of chromosome 5 LG3; scaffold01526; 157000374-469077464 of chromosome 6 LG2; scaffold01649; or 750954-487220871 of chromosome 7 LG7 of a *Pisum sativum* genome.

7. The method of claim 6, wherein the genomic region corresponds to a genomic region between positions: 386683682-424059129 of chromosome 2; 57407964-130197960 of chromosome 5; 541653217-end of chromosome 5; 387250594-427410060 of chromosome 2; 547677746-end of chromosome 5; start-17888751 of chromosome 6; 354324649-402078389 of chromosome 2; 18873917-69320372 of chromosome 3; 273327332-422042733 of chromosome 3; 118944413-200876233 of chromosome 4; 17641704-56905305 of chromosome 6; 79787890-153689044 of chromosome 7; 91738904-165762735 of chromosome 7; 207998578-360550624 of chromosome 7; scaffold00011; 73778015-347862320 of chromosome 1 LG6; 287196406-405820442 of chromosome 2 LG1; scaffold05541; 22316149-374998060 of chromosome 4 LG4; 35018291-496475925 of chromosome 5 LG3; 4102 if scaffold00839; 332130985-426328693 of chromosome 6 LG2; 8316115-481251260 of chromosome 7 LG7; scaffold02229; 122338217-340372022 of chromosome 1 LG6; 12117951-41766109 of chromosome 2 LG1; 55052537-420398638 of chromosome 3 LG5; scaffold06018; scaffold03592; super-scaffold8606; 47145729-440853776 of chromosome 4 LG4; scaffold02661; 3579419-570360377 of chromosome 5LG3; super-scaffold9678; 2560210-348314640 of chromosome 6 LG2; 191955823-63397608 of chromosome 7 LG7; 63453702 of chromosome 1 LG6; 96628783-415226641 of chromosome 2 LG1; scaffold05147; 403366757 of chromosome 4 LG4; scaffold00254; 811842-294741093 of chromosome 3 LG5; scaffold03249; 173468835-440508849 of chromosome 4 LG4; 362378-229071963 of chromosome 5 LG3; scaffold01526; 157000374-469077464 of chromosome 6 LG2; scaffold01649; or 750954-487220871 of chromosome 7 LG7 of the *Pisum sativum* Cameor vla reference genome.

8. The method of claim 6 or 7, wherein said yield QTL comprises an SNP marker associated with high yield; said height QTL comprises an SNP marker associated with height; and said lodging QTL comprises an SNP marker associated with low lodging.

9. The method of claim 8, wherein the SNP marker is selected from the group consisting of yield chr2-2 (Ps02_417306163), yield chr5-1 (Ps05_117545329), yield chr5-2 (Ps05_604790207), lodging chr2-1 (Ps02_415727951), lodging chr5-1 (Ps05_608256598), lodging chr6-1 (Ps06_5054240), height chr2-2 (Ps2_390480167), height chr3-1 (Ps03_74158043), height chr3-2 (Ps03_496270063), height chr4-1 (Ps04_192115819), height chr6-1 (Ps06_38889673), height chr7-1 (Ps07_115631962), height chr7-2 (Ps07_129458029), height chr7-3 (Ps07_256289873), Ps01_12111263, Ps01_21627814, Ps01_80920094, Ps01_165828422, Ps01_172888276, Ps01_274311344, Ps02_277385615, Ps01_306049485, Ps01_345383217, Ps01_414516587, Ps02_389051201, Ps02_406013484, Ps02_415945622, Ps03_98286373, Ps04_33913844, Ps04_383816669, Ps04_445153308, Ps05_48702172, Ps05_237258346, Ps05_318938718, Ps05_320108884, Ps05_526760209, Ps05_536892706, Ps06_32259152, Ps06_43753843, Ps06_105337899, Ps06_127479778, Ps06_367630606, Ps06_427519500, Ps07_9801763, Ps07_39866790, Ps07_129103458, Ps07_142955054, Ps07_173064631, Ps07_173321635, Ps07_278403545, Ps07_303005109, Ps07_481516842, Ps01_279286967, Ps01_405953972, Ps02_66314510, Ps02_146578429, Ps03_114813976, Ps03_120645295, Ps03_230771576, Ps03_253149185, Ps03_512230003, Ps03_531239107, Ps03_71303964, Ps04_69656387, Ps04_144177304, Ps04_156326296, Ps04_260958274, Ps04_264937409, Ps04_374148702, Ps04_453044385, Ps05_3468595, Ps05_223782536, Ps05_352490739, Ps05_473638215, Ps05_481936646, Ps05_528041290, Ps05_551582581, Ps05_583707944, Ps05_612824899, Ps05_616716582, Ps06_12615016, Ps06_33377536, Ps06_349137016, Ps06_425993896, Ps07_133823647, Ps07_194142598, Ps07_195412515, Ps01_68070331, Ps02_233036770, Ps02_342684459, Ps02_426672885, Ps02_433458109, Ps03_94359486, Ps03_104956337, Ps03_118892995, Ps03_195398925, Ps03_205809630, Ps03_241957626, Ps03_242777502, Ps03_251323511, Ps03_374110266, Ps03_437895589, Ps03_493092288, Ps04_205866569, Ps04_389245228, Ps04_430139407, Ps04_448803743, Ps05_5262178, Ps05_160013078, Ps05_174010831, Ps05_277684648, Ps06_213382570, Ps06_236678213, Ps06_398588276, Ps06_472058597, Ps06_478057404, Ps07_58281807, Ps07_105730908, Ps07_112377551, Ps07_114916793, Ps07_213648906, Ps07_234681662, Ps07_238767894, and Ps07_489306364.

10. The method of claim 9, wherein the SNP marker is selected from the group consisting of: T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A or G at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; G at position 195183596 of chromosome 7LG7; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of the *Pisum sativum* genome such as the *Pisum sativum* Cameor v1a reference genome.

11. A population of pea plants or seeds produced by the method of any one of claims 1-10, wherein the population of pea plants or seeds comprises a greater yield, greater height, and/or less lodging phenotype relative to a control population of pea plants or seeds lacking the one or more yield, height, and/or lodging alleles.

12. The population of pea plants or seeds of claim 11, wherein the population of pea plants or seeds (i) has average yield that is greater by at least 13 bushels per acre, average height that is greater by at least 8 inches, and/or average lodging that is lower by at least 1.5 lodging units relative to a control population of pea plants or seeds without said one or more yield, height, and/or lodging alleles; or (ii) comprises average protein and/or average oil content that is at least 90% relative to a control population of pea plants or seeds without said one or more yield, height, and/or lodging alleles.

13. A nucleic acid molecule for detecting a yield, height, and/or lodging molecular marker in a *Pisum sativum* genome, wherein the nucleic acid molecule comprises at least 15 nucleotides that include or are immediately adjacent to the marker, wherein the nucleic acid molecule is at least 90 percent identical to a sequence of the same number of consecutive nucleotides in either strand of DNA that include or are immediately adjacent to the marker.

14. The nucleic acid molecule of claim 13, wherein the yield, height, and/or lodging molecular marker is an SNP marker, and wherein the SNP marker is selected from the group consisting of T at position 407448748 of chromosome 2; T at position 4731 of scaffold 01713; a T at position 101 of SEQ ID NO: 1 in a genomic region comprising the nucleic acid sequence of SEQ ID NO: 1, or at a corresponding position of a genomic region at least 50 nucleotides of which is aligned to SEQ ID NO: 1 for at least 90% sequence identity; A at position 78866145 of chromosome 5; C at position 563627520 of chromosome 5; C at position 409407644 of chromosome 2; C at position 101 of SEQ ID NO: 4 in a genomic region comprising the nucleic acid sequence of SEQ ID NO: 4, or a corresponding position of a genomic region at least 50 nucleotides of which is aligned to SEQ ID NO: 4 for at least 90% sequence identity; G at position 566655216 of chromosome 5; G at position 6011352 of chromosome 6; G at position 101 of SEQ ID NO: 6 in a genomic region comprising the nucleic acid sequence of SEQ ID NO: 6, or a corresponding position of a genomic region at least 50 nucleotides of which is aligned to SEQ ID NO: 6 for at least 90% sequence identity; C or T at position 384481633 of chromosome 2LG1; C or T at position 101 of SEQ ID NO: 7 in a genomic region comprising the nucleic acid sequence of SEQ ID NO: 7, or a corresponding position of a genomic region at least 50 nucleotides of which is aligned to SEQ ID NO: 7 for at least 85% sequence identity; G or A at position 35492344 of chromosome 3; G or A at position 74088 of scaffold 02911; G or A at position 101 of SEQ ID NO: 8 in a genomic region comprising the nucleic acid sequence of SEQ ID NO: 8, or at a corresponding position of a genomic region at least 50 nucleotides of which is aligned to SEQ ID NO: 8 for at least 90% sequence identity; A or G at position 404873882 of chromosome 3LG5; T or C at position 158451622 of chromosome 4; T or C at position 74487 of scaffold 03924; T or C at position 101 of SEQ ID NO: 10 in a genomic region comprising the nucleic acid sequence of SEQ ID NO: 10, or at a corresponding position of a genomic region at least 50 nucleotides of which is aligned to SEQ ID NO: 10 for at least 90% sequence identity; T or C at position 31021563 of chromosome 6LG2; A or G at position 115361106 of chromosome 7LG7; G or A at position 131513318 of chromosome 7LG7; G or A at position 101 of SEQ ID NO: 13 in a genomic region comprising the nucleic acid sequence of SEQ ID NO: 13, or a corresponding position of a genomic region at least 50 nucleotides of which is aligned to SEQ ID NO: 13 for at least 90% sequence identity; A or G at position 324031049 of chromosome 7; A or G at position 13857 of scaffold 04080; A or G at position 101 of SEQ ID NO: 14 in a genomic region comprising the nucleic acid sequence of SEQ ID NO: 14, or at a corresponding position of a genomic region at least 50 nucleotides of which is aligned to SEQ ID NO: 14 for at least 90% sequence identity; T at position 17194777 of chromosome 1, T at position 128561964 of chromosome 4LG4; T at position 101 of SEQ ID NO: 15 in a genomic region comprising the nucleic acid sequence of SEQ ID NO: 15, or at a corresponding position of a genomic region at least 50 nucleotides of which is aligned to SEQ ID NO: 15 for at least 90% sequence identity; T at position 24551584 of chromosome 1; T at position 4366 of scaffold 00011; T at position 101 of SEQ ID NO: 16 in a genomic region comprising the nucleic acid sequence of SEQ ID NO: 16, or at a corresponding position of a genomic region at least 50 nucleotides of which is aligned to SEQ ID NO: 16 for at least 90% sequence identity; T at position 73778015 of chromosome 1LG6; G at position 150246299 of chromosome 1LG6; G at position 151466935 of chromosome 1LG6; A at position 214738954 of chromosome 1LG6; T at position 287196406 of chromosome 2LG1; G at position 244424866 of chromosome 1; G at position 12681 of scaffold 05541; G at position 101 of SEQ ID NO: 22 in a genomic region comprising the nucleic acid sequence of SEQ ID NO: 22, or at a corresponding position of a genomic region at least 50 nucleotides of which is aligned to SEQ ID NO: 22 for at least 90% sequence identity; C at position 290859608 of chromosome 1LG6; C at position 347862320 of chromosome 1LG6; G at position 383268719 of chromosome 2LG1; C at position 397000005 of chromosome 2LG1; G at position 405820442 of chromosome 2LG1; A at position 55052537 of chromosome 3LG5; T at position 22316149 of chromosome 4LG4; G at position 398264448 of chromosome 4LG4; C at position 374998060 of chromosome 4LG4; A at position 35018291 of chromosome 5LG3; G at position 190837200 of chromosome 5LG3; G at position 12480905 of chromosome 5LG3; T at position 268153146 of chromosome 5LG3; A at position 489005341 of chromosome 5LG3; T at position 496475925 of chromosome 5LG3; A at position 12702042 of chromosome 6; A at position 4102 of scaffold 00839; A at position 101 of SEQ ID NO: 38 in a genomic region comprising the nucleic acid sequence of SEQ ID NO: 38, or at a corresponding position of a genomic region at least 50 nucleotides of which is aligned to SEQ ID NO: 38 for at least 90% sequence identity; G at position 332130985 of chromosome 6LG2; T at position 56896622 of chromosome 6LG2; A at position 112269847 of chromosome 6LG2; C at position 368401417 of chromosome 6LG2; G at position 426328693 of chromosome 6LG2; A at position 8316115 of chromosome 7LG7; T at position 37445789 of chromosome 7LG7; A at position 132484304 of chromosome 7LG7; A at position 144420546 of chromosome 7LG7; C at position 172275819 of chromosome 7LG7; G at position 16557144 of chromosome 7LG7; C at position 285299600 of chromosome 7; C at position 22321 of scaffold 02229; C at position 101 of SEQ ID NO: 50 in a genomic region comprising the nucleic acid sequence of SEQ ID NO: 50, or at a corresponding position of a genomic region at least 50 nucleotides of which is aligned to SEQ ID NO: 50 for at least 90% sequence identity; G at position 359039170 of chromosome 7LG7; T at position 481251260 of chromosome 7LG7; A at position 122338217 of chromosome 1LG6; C at position 340372022 of chromosome 1LG6; T at position 41766109 of chromosome 2LG1; G at position 12117951 of chromosome 2LG1; A at position 67204106 of chromosome 3LG5; C at position 73696006 of chromosome 3; C at position 3352 of scaffold 06018; C at position 101 of SEQ ID NO: 58 in a genomic region comprising the nucleic acid sequence of SEQ ID NO: 58, or at a corresponding position of a genomic region at least 50 nucleotides of which is aligned to SEQ ID NO: 58 for at least 90% sequence identity; C at position 166350011 of chromosome 3LG5; C at position 187378726 of chromosome 3; C at position 85406 of scaffold 03592; C at position 101 of SEQ ID NO: 60 in a genomic region comprising the nucleic acid sequence of SEQ ID NO: 60, or at a corresponding position of a genomic region at least 50 nucleotides of which is aligned to SEQ ID NO: 60 for at least 90% sequence identity; G at position 420398638 of chromosome 3LG5; C at position 425968088 of chromosome 3; C at position 563531892 of chromosome 5LG3; C at position 101 of SEQ ID NO: 62 in a genomic region comprising the nucleic acid sequence of SEQ ID NO: 62, or at a corresponding position of a genomic region at least 50 nucleotides of which is aligned to SEQ ID NO: 62 for at least 90% sequence identity; G at position 31363236 of chromosome 3; G at position 249079 of super-scaffold 8606; G at position 101 of SEQ ID NO: 63 in a genomic region comprising the nucleic acid sequence of SEQ ID NO: 63, or at a corresponding position of a genomic region at least 50 nucleotides of which is aligned to SEQ ID NO: 63 for at least 90% sequence identity; A at position 47145729 of chromosome 4LG4; C at position 119133416 of chromosome 4LG4; G at position 130758952 of chromosome 4LG4; C at position 228877652 of chromosome 4; C at position 83020 of scaffold 02661; C at position 101 of SEQ ID NO: 67 in a genomic region comprising the nucleic acid sequence of SEQ ID NO: 67, or at a corresponding position of a genomic region at least 50 nucleotides of which is aligned to SEQ ID NO: 67 for at least 90% sequence identity; C at position 232585904 of chromosome 4; C at position 403189011 of chromosome 3LG5; C at position 101 of SEQ ID NO: 68 in a genomic region comprising the nucleic acid sequence of SEQ ID NO: 68, or at a corresponding position of a genomic region at least 50 nucleotides of which is aligned to SEQ ID NO: 68 for at least 90% sequence identity; T at position 285109838 of chromosome 4LG4; T at position 440853776 of chromosome 4LG4; A at position 3579419 of chromosome 5LG3; A at position 187232313 of chromosome 5LG3; A at position 84459119 of chromosome 5LG3; C at position 438580908 of chromosome 5LG3; T at position 437711160 of chromosome 5; T at position 10598 of super-scaffold9678; T at position 101 of SEQ ID NO: 75 in a genomic region comprising the nucleic acid sequence of SEQ ID NO: 75, or at a corresponding position of a genomic region at least 50 nucleotides of which is aligned to SEQ ID NO: 75 for at least 90% sequence identity; G at position 489523514 of chromosome 5LG3; G at position 509926470 of chromosome 5LG3; T at position 543282804 of chromosome 5LG3; T at position 570360377 of chromosome 5LG3; C at position 8937964 of chromosome 5LG3; A at position 11907302 of chromosome 6LG2; A at position 2560210 of chromosome 6LG2; G at position 348314640 of chromosome 6LG2; C at position 185310300 of chromosome 6LG2; G at position 137600708 of chromosome 7; G at position 112005091 of chromosome 2LG1; T at position 101 of SEQ ID NO: 85 in a genomic region comprising the nucleic acid sequence of SEQ ID NO: 85, or at a corresponding position of a genomic region at least 50 nucleotides of which is aligned to SEQ ID NO: 85 for at least 90% sequence identity; A at position 191955823 of chromosome 7LG7; G at position 195183596 of chromosome 7LG7; A or T at position 63453702 of chromosome 1LG6; T or C at position 96628783 of chromosome 2LG1; C or T at position 341987428 of chromosome 2LG1; C or G at position 415226641 of chromosome 2LG1; C or T at position 425305138 of chromosome 2; C or T at position 6226 of scaffold05147; C or T at position 101 of SEQ ID NO: 92 in a genomic region comprising the nucleic acid sequence of SEQ ID NO: 92, or at a corresponding position of a genomic region at least 50 nucleotides of which is aligned to SEQ ID NO: 92 for at least 90% sequence identity; C or T at position 52540141 of chromosome 3LG5; G or A at position 60235545 of chromosome 3; G or A at position 63397608 of chromosome 7LG7; G or A at position 101 of SEQ ID NO: 94 in a genomic region comprising the nucleic acid sequence of SEQ ID NO: 94, or at a corresponding position of a genomic region at least 50 nucleotides of which is aligned to SEQ ID NO: 94 for at least 90% sequence identity; A or T at position 72192607 of chromosome 3; A or T at position 403366757 of chromosome 4LG4; A or T at position 101 of SEQ ID NO: 95 in a genomic region comprising the nucleic acid sequence of SEQ ID NO: 95, or at a corresponding position of a genomic region at least 50 nucleotides of which is aligned to SEQ ID NO: 95 for at least 90% sequence identity; T or G at position 137302656 of chromosome 3LG5; A or C at position 146763395 of chromosome 3; A or C at position 82633 of scaffold00254; A or C at position 101 of SEQ ID NO: 97 in a genomic region comprising the nucleic acid sequence of SEQ ID NO: 97, or at a corresponding position of a genomic region at least 50 nucleotides of which is aligned to SEQ ID NO: 97 for at least 90% sequence identity; T or A at position 175749690 of chromosome 3LG5; C or T at position 811842 of chromosome 3LG5; A or G at position 182785714 of chromosome 3LG5; G or A at position 289130533 of chromosome 3LG5; C or T at position 294741093 of chromosome 3LG5; C or G at position 393239734 of chromosome 3; C or G at position 112747 of scaffold03249; C or G at position 101 of SEQ ID NO: 103 in a genomic region comprising the nucleic acid sequence of SEQ ID NO: 103, or at a corresponding position of a genomic region at least 50 nucleotides of which is aligned to SEQ ID NO: 103 for at least 90% sequence identity; T or G at position 173468835 of chromosome 4LG4; G or A at position 402922728 of chromosome 4LG4; T or C at position 435276132 of chromosome 4LG4; G or A at position 440508849 of chromosome 4LG4; C or T at position 362378 of chromosome 5LG3; A or G at position 122579305 of chromosome 5LG3; A or C at position 142238758 of chromosome 5; A or C at position 123477 of scaffold01526; A or C at position 101 of SEQ ID NO: 110 in a genomic region comprising the nucleic acid sequence of SEQ ID NO: 110, or at a corresponding position of a genomic region at least 50 nucleotides of which is aligned to SEQ ID NO: 110 for at least 90% sequence identity; T or A at position 229071963 of chromosome 5LG3; G or T at position 157000374 of chromosome 6LG2; G or A at position 169305572 of chromosome 6LG2; G or A at position 396152218 of chromosome 6LG2; T or C at position 469077464 of chromosome 6LG2; C or T at position 471433793 of chromosome 6; C or T at position 134790 of scaffold01649; C or T at position 101 of SEQ ID NO: 116 in a genomic region comprising the nucleic acid sequence of SEQ ID NO: 116, or at a corresponding position of a genomic region at least 50 nucleotides of which is aligned to SEQ ID NO: 116 for at least 90% sequence identity; A or G at position 56384258 of chromosome 7LG7; G or C at position 104698039 of chromosome 7LG7; A or G at position 45871371 of chromosome 7LG7; A or G at position 116467927 of chromosome 7LG7; G or A at position 750954 of chromosome 7LG7; C or T at position 293412012 of chromosome 7LG7; T or G at position 310516174 of chromosome 7LG7; and A or G at position 487220871 of chromosome 7LG7 of the *Pisum sativum* genome.

15. The nucleic acid molecule of claim 13 or 14, further comprising a detectable label.
